# FASCICULE DE BREVET EUROPEEN

(11) **EP 2 335 683 B1**
(45) Date de publication et mention de la délivrance du brevet: **28.02.2018**
(21) Numéro de dépôt: 10194667.1
(22) Date de dépôt: 13.12.2010
(51) Int. Cl.: A61K 8/81, A61K 8/92, A61K 8/86, A61Q 19/00

(54) **Kit de formulation d'un produit cosmétique**
Formulierungskit eines Kosmetikprodukts
Cosmetic kit formulation

(30) Priorité: 16.12.2009 FR 0959051
(43) Date de publication de la demande: 22.06.2011
(73) Titulaire: L'Oréal, 75008 Paris (FR)
(72) Inventeur: Cotton, Thierry, 75013, Paris (FR); Labatut, Frédérique, 75014, Paris (FR); Rieffel, Maud, 77515, Pommeuse (FR); Martin, Guénaelle, 92290, Chatenay Malabry (FR); Levy, Florence, 75011, Paris (FR)
(74) Mandataire: Duvert, Sandra

(56) Documents cités:
- EP-A1- 1 704 896
- EP-A1- 2 243 472
- FR-A1- 2 886 845
- US-A1- 2002 193 321
- US-A1- 2006 140 895

## Description

La présente invention a trait à des kits de formulation de produits cosmétiques, et en particulier à un kit de formulation de produits cosmétiques sous forme d'émulsion comprenant deux compositions, les compositions étant destinées à être mélangés extemporanément pour former un produit cosmétique sous forme d'émulsion.

L'invention se rapporte aussi à un procédé de traitement cosmétique des matières kératiniques telles que la peau, les muqueuses, les fibres kératiniques telles que les cheveux, mettant en oeuvre lesdites compositions.

Les utilisateurs souhaitent de plus en plus disposer d'une composition « personnalisée », adaptée à l'usage qu'ils veulent en faire au moment où ils en disposent. Ainsi, selon l'humeur, les saisons, la température extérieure ou encore la problématique de peau, l'utilisateur aspire à disposer d'une composition adaptée à son besoin du moment, par exemple disposer d'une composition plus ou moins épaisse ou plus ou moins concentrée en actif ou ayant une coloration ou un parfum particulier. Les consommateurs recherchent en particulier des produits cosmétiques personnalisé qu'ils peuvent préparer eux même à la maison.

De plus, ce type de galénique présente l'avantage de pouvoir diminuer ou supprimer les conservateurs qui sont indispensables à la bonne conservation d'un produit cosmétique conventionnel et de pouvoir proposer un gamme de principe actifs hydrophile ou lipophile fragiles qu'une formulation « fraîche » permet de stabiliser (grâce à une durée de conservation et des conditions de stockage spécifiques).

On connaît des kits destinés à la préparation extemporanée de compositions cosmétiques personnalisées, qui comprennent plusieurs compositions séparées, et notamment une première composition dite « de base » sous forme d'émulsion et des compositions additionnelles conditionnées séparément comprenant des actifs cosmétiques, des parfums ou des colorants, permettant à la consommatrice de personnaliser la composition de base en fonction de ses besoins.
Ces systèmes mettent donc en oeuvre des émulsions déjà formées dans lesquelles on ajoute en plusieurs étapes des additifs cosmétiques variés.

Ces produits à préparer « à la maison » ne présentent pas toujours des qualités cosmétiques optimales, équivalentes aux produits finis disponibles dans le commerce, notamment en terme de texture.

Il subsiste donc le besoin de systèmes permettant de réaliser facilement (sans appareillage particulier), et rapidement (en un nombre réduit d'étapes), des produits sous forme complexe telle qu'une émulsion présentant une bonne cosméticité, comparable aux produits cosmétiques conventionnels prêts à l'utilisation disponibles dans le commerce.

L'invention a ainsi pour objet un procédé de traitement cosmétiques des matières kératiniques consistant à :
- disposer d'une composition A comprenant au moins une huile, au moins un agent gélifiant se présentant sous forme dispersée dans un solvant organique ou aqueux, et au moins un tensioactif non ionique choisi parmi les esters de polyols et d'acide gras à chaîne saturée ou insaturée comportant de 8 à 24 atomes de carbone, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre et d'acide gras en C₈-C₂₄, et leurs dérivés oxyalkylénés; les éthers d'alcools gras; les éthers de sucre et d'alcools gras en C₈-C₂₄, et leurs mélanges, ladite composition comprenant moins de 10% en poids d'eau
- disposer d'une composition B aqueuse comprenant exclusivement de l'eau et éventuellement au moins un solvant organique hydrosoluble,
- les compositions A et B étant conditionnées séparément,
- mélanger par agitation manuelle de façon extemporanée les compositions A et B de manière à former une émulsion,
puis à appliquer le mélange sur les matières kératiniques.

L'invention a également pour objet un procédé de traitement cosmétiques des matières kératiniques consistant à :
- disposer d'une composition A comprenant au moins une huile, au moins un agent gélifiant se présentant sous forme dispersée dans un solvant organique ou aqueux, et au moins un tensioactif non ionique choisi parmi les esters de polyols et d'acide gras à chaîne saturée ou insaturée comportant de 8 à 24 atomes de carbone, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre et d'acide gras en C₈-C₂₄, et leurs dérivés oxyalkylénés; les éthers d'alcools gras; les éthers de sucre et d'alcools gras en C₈-C₂₄, et leurs mélanges, ladite composition comprenant moins de 10% en poids d'eau et une teneur en huile allant de 30% à 95% en poids
- disposer d'une composition B aqueuse ,
- les compositions A et B étant conditionnées séparément,
- mélanger par agitation manuelle de façon extemporanée les compositions A et B de manière à former une émulsion,
puis à appliquer le mélange sur les matières kératiniques.

Les compositions A et B sont mélangées extemporanément en vue de former un produit cosmétique sous forme d'émulsion.

Ce procédé permet de réaliser en un nombre réduit d'étapes (en particulier en une seule étape), sans apport d'énergie et par simple agitation manuelle, une émulsion présentant une texture cosmétique, stable.

Le produit cosmétique sous forme d'émulsion peut être utilisé notamment comme produit de soin ou de maquillage des matières kératiniques, ou encore un produit de nettoyage ou de démaquillage des matières kératiniques.

Le mélange extemporané est obtenu par simple mélange des quantités voulues (en général pré déterminées) des compositions A et B, ces quantités étant déterminées selon le but final recherché.
La dose appropriée de composition A et de composition B peut être obtenue en utilisant des formes de présentation en mono-dose, telles que des sachets, des bouillotes, des tubes, des ampoules, des seringues pré-remplies, des capsules molles, des coques ou barquettes en plastique thermoformé. Une dose appropriée peut également être obtenue à partir d'une présentation multi-doses en utilisant un système distribuant une dose pré-définie. Un tel système peut être un flacon pompe, un aérosol, une pipette ou une seringue graduée, un compte-gouttes.
En particulier, les compositions A et B peuvent être conditionnées sous forme de sachet, de bouillotte ou de pot.

Le produit cosmétique final est obtenu par mélange extemporané de quantités appropriées de la composition A et de la composition B.
Ledit produit cosmétique est de préférence obtenu en ajoutant la composition A à la composition B sous mélange.
Le produit final est une émulsion, de préférence une émulsion huile dans eau. Selon un mode de réalisation avantageux, la composition A représente de 2 à 70% en poids, de préférence de 10 à 60% en poids, encore mieux de 20 à 50% en poids par rapport au poids total du produit cosmétique final.

L'invention a aussi pour objet un kit de formulation d'un produit cosmétique sous forme d'émulsion comprenant :
- une composition A comprenant au moins une huile, au moins un agent gélifiant se présentant sous forme dispersée dans un solvant organique ou aqueux et au moins un tensioactif non ionique choisi parmi les esters de polyols et d'acide gras à chaîne saturée ou insaturée comportant de 8 à 24 atomes de carbone, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre et d'acide gras en C₈-C₂₄, et leurs dérivés oxyalkylénés; les éthers d'alcools gras; les éthers de sucre et d'alcools gras en C₈-C₂₄, et leurs mélanges, ladite composition comprenant moins de 10% en poids d'eau et une teneur en huile allant de 30% à 95% en poids,
- une composition B aqueuse comprenant exclusivement de l'eau et éventuellement au moins un solvant organique hydrosoluble,
les compositions A et B étant conditionnées séparément.

Ainsi, la demande divulgue également un kit de formulation d'un produit cosmétique sous forme d'émulsion comprenant :
- une composition A comprenant au moins une huile, au moins un agent gélifiant se présentant sous forme dispersée dans un solvant organique ou aqueux, ladite composition comprenant moins de 10% en poids d'eau, et
- une composition B aqueuse,
les compositions A et B étant conditionnées séparément et la composition A représentant de 2 à 70% en poids, de préférence de 10 à 60% en poids, encore mieux de 20 à 50% en poids par rapport au poids total des compositions A et B.

Selon un mode de réalisation, le kit de formulation selon l'invention comprend, outre les compositions A et B, un moyen pour mélanger les compositions A et B tel qu'une spatule et/ou un récipient tel qu'un pot destiné à contenir le produit final sous forme d'émulsion formé par le mélange des compositions A et B.

Selon un mode de réalisation, le kit selon l'invention peut comprendre en outre des instructions, notamment sur une notice explicative, pour préparer le produit cosmétique.
Le mélange des compositions A et B se fait sous simple agitation manuelle, sans autre apport d'énergie, notamment sans chauffage, une émulsion lisse et homogène se forme rapidement. De préférence, on ajoute la composition A à la composition B.

Le produit final sous forme d'émulsion peut être conservé au frais après fabrication, notamment à une température allant de 2 à 10°C, par exemple au réfrigérateur, pour une durée allant par exemple de 1 à 8 semaines.

Selon un mode de réalisation, la consommatrice utilise comme composition aqueuse B de l'eau minérale disponible dans le commerce.

Les kit et procédé selon l'invention peuvent permettre de moduler extemporanément par l'utilisateur, la composition, la rhéologie, la couleur ou la concentration en actif du produit final.

En outre, dans le cas de l'application d'actifs sensibles à un stimulus extérieur, leur incorporation dans des produits fabriqués extemporanément par simple mélange et conservés au frais pour une durée limitée permet de garantir leur efficacité et/ou leur stabilité.
Ces conditions de fabrication et de conservation permettent également de diminuer ou supprimer les agents conservateurs. Ainsi selon un mode de réalisation, les compositions A et B et le produit cosmétique issu du mélange desdites compositions comprennent moins de 1% d'agent conservateurs, voire sont exempts d'agents conservateurs.

D'autres objets encore apparaîtront dans la description détaillée qui suit.

### I/ Composition A

La composition A comprend moins de 10% en poids d'eau, de préférence moins de 5% d'eau, mieux moins de 3% en poids d'eau, encore mieux moins de 1% en poids d'eau Avantageusement, la composition A est anhydre et ne comprend pas d'eau ajoutée, autre que l'eau apportée via le biais d'une ou plusieurs matières premières constituant ladite composition.

### Huile

La composition A comprend au moins une huile (corps gras liquide à température ambiante (20-25°C)) qui forme en totalité ou en partie la phase grasse de la composition. L'huile peut être choisie parmi les huiles volatiles ou non volatiles, d'origine végétale, minérale ou synthétique, et leurs mélanges. Ces huiles sont physiologiquement acceptables.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité;
- les esters et les éthers de synthèse, notamment d'acides gras :
   les esters gras peuvent être choisis par exemple parmi ceux obtenus à partir d'un alcool à chaîne linéaire, ou ramifiée, saturée ou insaturée ayant de 1 à 24 atomes de carbone et d'un acide gras à chaîne linéaire ou ramifiée, ayant de 3 à 24 atomes de carbone. Comme esters gras, on peut citer par exemple le caprate/caprylate d'éthyl-2 hexyle (ou caprate/caprylate d'octyle), le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le myristate d'octyl-2 dodécyle, le monococoate d'éthyl-2 hexyle (ou monococoate d'octyle), le palmitate de méthyle, le palmitate d'éthyle, le palmitate d'isopropyle, le palmitate d'isobutyle, palmitate d'ethyl-2 hexyle (ou palmitate d'octyle), le stéarate de butyle, le stéarate d'isopropyle, le stéarate d'isobutyle, le stéarate d'éthyl-2 hexyle (ou stéarate d'octyle), l'isostéarate d'isopropyle, le stéarate d'isocétyle, l'isostéarate d'isotéaryle, le pelargonate d'ethyl-2 hexyle (ou pelargonate d'octyle), l'hydroxystéarate d'éthyl-2 hexyle (ou hydroxystéarate d'octyle), le decyl oleate, l'adipate de di-isopropyle, l'adipate de di-éthyl-2 hexyle (ou adipate de di-octyle), l'adipate de diisocetyle, le succinate d'ethyl-2 hexyle (ou succinate d'octyle), le sébacate de diisopropyle, le malate d'ethyl-2 hexyle (ou malate d'octyle), le caprate/caprylate de pentaérythritol, l'hexanoate d'éthyl-2 hexyle (ou hexanoate d'octyle), l'octanoate d'octyldodécyle, le néopentanoate d'isodécyle, le néopentanoate d'isostéaryle, le neopentanoate d'octyldodecyle, l'isononanoate d'isononyle, l'isononanoate d'isotridécyle, l'isononanoate de cétéaryle, l'isononanoate d'isodécyle, l'isononanoate d'isotridécyle, le lactate de lauryle, le lactate de myristyle, le lactate de cétyle, le propionate de myristyle, l'éthyl-2 hexanoate d'éthyl-2 hexyle (ou éthyl-2 hexanoate d'octyle), l'octanoate d'éthyl-2 hexyle (ou octanoate d'octyle), l'ethyl-2 hexanoate de cetyle, le pentaérythritol de tétraisostéarate, le lauroyl sarcosinate d'isopropyle (Eldew SL 205 de de la société Unipex), le dicaprylyl carbonate (Cetiol CC de la société Cognis), le benzoates d'alcools gras en C12-C15 (Finsolv TN de la société FINETEX).
      Comme éthers gras, on peut citer le Dicaprylyl ether (Cetiol OE de la société Cognis).
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les alcanes linéaires, de préférence d'origine végétale, comprenant de 7 à 19 atomes de carbone, en particulier de 9 à 17 atomes de carbone, et plus particulièrement de 11 à 14 atomes de carbone. Ils peuvent présenter un point éclair compris dans l'intervalle variant de 30 à 120 °C, et plus particulièrement de 40 à 100 °C et sont de préférence liquides à température ambiante (environ 25 °C) et à la pression atmosphérique (760 mmHg).

A titre d'exemple d'alcane linéaire convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcane linéaire convenant à l'invention, on peut citer le n-heptane (C7), le n-octane (C8), le n-nonane (C9), le n-décane (C10), le n-undécane (C11), le n-dodécane (C12), le n-tridécane (C13), le n-tétradecane (C14), le -pentadécane (C15), le n-hexadécane (C16), le n-heptadécane (C17), le n-octadécane (C18), le -nonadécane (C19) et leurs mélanges, et en particulier le mélange de n-undécane (C11) et de n-tridécane (C13) décrit à l'exemple 1 de la demande WO2008/155059 de la Société Cognis. On peut également citer le n-dodécane (C12) et le n-tétradécane (C14) vendus par Sasol respectivement sous les références PARAFOL 12-97 et PARAFOL 14-97, ainsi que leurs mélanges.

On pourra utiliser l'alcane linéaire seul ou en mélange d'au moins deux alcanes distincts et différant entre eux d'un nombre de carbone d'au moins 1, et notamment un mélange d'au moins deux alcanes linéaires comportant de 10 à 14 atomes de carbone distincts et différant entre eux d'un nombre de carbone d'au moins 2, et en particulier un mélange d'alcanes linéaires volatiles C11/C13 ou un mélange d'alcanes linéaires C12/C14, en particulier un mélange n-undécane/n-tridécane (un tel mélange peut être obtenu selon l'exemple 1 ou l'exemple 2 du WO 2008/155059) ;Ce type d'huiles s'étalent facilement sur la peau et laissent un toucher non gras et non collant,
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; les polydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On peut citer en particulier les huiles choisies parmi les huiles végétales ou d'origine végétale.

Dans un mode de réalisation, l'huile est de préférence présente en teneur allant de 30% à 95% en poids par rapport au poids total de la composition A, de préférence de 40 à 90% en poids, et mieux de 40 à 80% en poids.

La phase grasse de la composition peut comprendre aussi tout additif habituel liposoluble ou lipodispersible comme par exemple d'autres corps gras tels que des cires, des composés pâteux, des alcools gras, des acides gras.

### Gélifiant

L'agent gélifiant (ou épaississant) utilisé dans la composition A du kit selon l'invention se présente sous forme dispersée dans un solvant organique et/ou aqueux. Il est ainsi apte à gonfler et à épaissir rapidement la composition lorsqu'il est mis en contact avec l'eau apportée par la composition B.
De préférence l'agent gélifiant est choisi parmi les polymères se présentant sous forme d'émulsion inverse eau-dans-huile.
La quantité (en matière active) d'agent gélifiant peut aller de 0,1% à 25% en poids, de préférence de 0,5% à 20% en poids et encore mieux de 1% à 15% en poids par rapport au poids total de la composition A.

L'agent gélifiant est de préférence un polymère qui peut être choisi notamment parmi i) les copolymères d'acide carboxylique à insaturation α,β-monoéthylénique, notamment d'acide acrylique ou méthacrylique, ii) les copolymères à base d'acide 2-acrylamido-2-méthylpropane sulfonique et leurs mélanges.

De façon préférentielle, les polymères gélifiants conformes à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés », des polymères totalement ou pratiquement totalement neutralisés, c'est-à-dire neutralisés à au moins 90%.

On entend par « copolymères » aussi bien les copolymères obtenus à partir de deux sortes de monomères que ceux obtenus à partir de plus de deux sortes de monomères tels que les terpolymères obtenus à partir de trois sortes de monomères.
i) Les copolymères d'acide carboxylique à insaturation α,β-monoéthylénique, notamment d'acide acrylique ou méthacrylique sont des copolymères obtenus par copolymérisation d'un ou plusieurs monomères (a) choisis parmi les acides carboxyliques à insaturation α,β-éthylénique ou leurs esters, avec un monomère (b) à insaturation éthylénique comportant un groupement hydrophobe.
   L'acide carboxylique à insaturation α,β-monoéthylénique constituant le monomère (a) peut être choisi parmi de nombreux acides et en particulier parmi l'acide acrylique, l'acide méthacrylique, l'acide crotonique, l'acide itaconique, l'acide maléique, et leurs esters. Il s'agit de préférence de l'acide acrylique ou de l'acide méthacrylique, et leurs esters.
   Le monomère (b) comportant un groupement hydrophobe peut être en particulier choisi parmi les acrylates, méthacrylates ou itaconates d'alcool gras en C₁₂-C₃₀ oxyéthyléné (1 à 50 OE), tels que le steareth-20 méthacrylate, le méthacrylate de béhényle oxyéthyléné (25 OE), l'itaconate de mono-cétyle oxyéthyléné (20 OE), l'itaconate de mono-stéaryle oxyéthyléné (20 OE), l'acrylate modifié par des alcools en C12-C24 polyoxyéthylénés (25 OE), et parmi les acrylates ou méthacrylates d'acide gras en C₁₂-C₃₀ tels que les acrylates ou méthacrylates de décyle, de lauryle, de stéaryle, de béhényle ou de mélissyle, et leurs mélanges.
   Comme polymères comportant au moins un monomère d'acrylates, méthacrylates ou itaconates d'alcool gras en C₁₂-C₃₀ oxyéthyléné, on peut citer notamment le copolymère acrylates/steareth-20 methacrylate sous forme de dispersion aqueuse, comme le produit commercialisé sous la dénomination Aculyn 22 par la société Rohm & Haas (nom CTFA : Acrylates/Steareth-30 Methacrylate copolymer) ; le terpolymère acide (méth)acrylique / acrylate d'éthyle / méthacrylate de béhényle oxyéthyléné (25 OE), tel que le produit en émulsion aqueuse commercialisé sous la dénomination Aculyn 28 par la société Rohm & Haas ; le copolymère acide acrylique/itaconate de mono-cétyle oxyéthyléné (20 OE), tel que le produit en dispersion aqueuse à 30 % commercialisé sous la dénomination Structure 3001 par la société National Starch ; le copolymère acide acrylique/itaconate de mono-stéaryle oxyéthyléné (20 OE) tel que le produit en dispersion aqueuse à 30 % commercialisé sous la dénomination Structure 2001 par la société National Starch ; le copolymère acrylates/acrylate modifié par des alcools en C12-C24 polyoxyéthylénés (25 OE), tel que le latex à 30-32 % de copolymère, commercialisé sous la dénomination Synthalen W2000 par la société 3V SA.
   Parmi ces polymères gélifiants, on utilise en particulier les copolymères comportant au moins un monomère d'acrylates, méthacrylates ou itaconates d'alcool gras en C₁₂-C₃₀ oxyéthyléné, préférentiellement, les copolymères comportant au moins un monomère d'acrylate, méthacrylate ou itaconate d'alcool gras en C₁₂-C₃₀ oxyéthyléné, notamment le copolymère acrylates/steareth-20 methacrylate, comme le copolymère acrylates/steareth-20 methacrylate (Aculyn 22).
ii) Les copolymères à base d'acide 2-acrylamido-2-méthylpropane sulfonique peuvent être choisis parmi les copolymère d'acide 2-acrylamido-2-méthylpropane sulfonique et de monomères hydrophiles à insaturation éthylénique choisis par exemple parmi les acides (méth)acryliques, leurs dérivés alkyl substitués en β ou leurs esters obtenus avec des monoalcools ou des mono- ou poly- alkylèneglycols, les alkyl(méth)acrylamides, la vinylpyrrolidone, le vinylformamide, l'anhydride maléique, l'acide itaconique, l'acide maléique ou les mélanges de ces composés.

Ces polymères selon l'invention peuvent être réticulés ou non réticulés.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Comme copolymère susceptible d'être obtenu à partir d'acide 2-acrylamido-2-méthylpropane sulfonique et de monomères hydrophiles à insaturation éthylénique, on peut utiliser notamment les copolymères d'acide 2-acrylamido-2-méthylpropane sulfonique et d'alkyl (méth)acrylamides comme par exemple :
(1) les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom C.T.F.A. : Polyacrylamide / C13-14 Isoparaffin / Laureth-7), sous le nom de SIMULGEL 600 par la société Seppic (nom C.T.F.A. : Acrylamide / Sodium acryloyldimethyltaurate copolymer / Isohexadecane / Polysorbate 80),
(2) les copolymères d'acide(méth)acrylique ou de (méth)acrylate et d'acide 2-acrylamido-2-méthylpropane sulfonique, notamment ceux se présentant sous la forme d'une émulsion E/H, tels que ceux commercialisés sous le nom de SIMULGEL NS par la société Seppic (copolymère d'acrylamido-2-methyl propane sulfonate de sodium / hydroxyethyl acrylate en émulsion inverse à 40% dans Polysorbate 60 et squalane) (nom CTFA : hydroxyethyl acrylate/sodium acryloyldimethyltaurate copolymer / squalane / polysorbate 60) ou ceux commercialisés sous le nom de SIMULGEL EG par la société Seppic (copolymère d'acide acrylique / acrylamido-2-methylpropane-sulfonique sous forme de sel de sodium en émulsion inverse à 45% dans isohexadecane / eau) (nom C.T.F.A. : Sodium Acrylate / Sodium acryloydimethyl-taurate copolymer / Isohexadecane / Polysorbate 80).

De préférence la composition A du kit selon l'invention comprend au moins un agent gélifiant choisi parmi les copolymères d'acide 2-acrylamido-2-méthylpropane sulfonique, en particulier les copolymères d'acide 2-acrylamido-2-méthylpropane sulfonique et d'alkyl(méth)acrylamide tels que décrits plus haut.
De préférence la composition A du kit selon l'invention comprend au moins un agent gélifiant choisi parmi les copolymères d'acide 2-acrylamido-2-méthylpropane sulfonique, en particulier les copolymères d'acide 2-acrylamido-2-méthylpropane sulfonique et d'alkyl(méth)acrylamide se présentant sous la forme d'une émulsion inverse eau-dans-huile, l'huile étant en particulier un alcane comprenant de 7 à 16 atomes de carbone, par exemple un alcane ramifié tel que l'isododécane ou l'isohexadécane, l'émulsion comprenant de préférence un tensioactif tel que le polyoxyethylene Sorbitan Monooleate (20 OE.).

### Tensioactifs

La composition A selon l'invention comprend au moins un tensioactif (ou émulsionnant). Les tensioactifs utilisés sont de préférence des tensioactifs aptes à former des émulsions sans apport de chaleur et par simple agitation manuelle.
Les tensioactifs peuvent être présents en une teneur allant 1% à 25% en poids, de préférence de 3% à 20% en poids et encore mieux de 5% à 15% en poids par rapport au poids total de la composition A.

Les tensioactifs peuvent être choisis parmi les tensioactifs amphotères, anioniques, cationiques ou non ioniques, utilisés seuls ou en mélange. Les émulsionnants sont choisis de manière appropriée suivant la phase continue de l'émulsion à obtenir (E/H ou H/E).

Comme tensioactifs utilisables pour la préparation des émulsions E/H, on peut citer par exemple les alkyl esters ou éthers de sorbitan, de glycérol ou de sucres.

On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Pour les émulsions H/E, la composition A comprend comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

Comme ester de glycéryle et d'acide gras, on peut citer notamment le stéarate de glycéryle (mono-, di- et/ou tri-stéarate de glycéryle) (nom CTFA : glyceryl stearate) ou le ricino-léate de glycéryle, et leurs mélanges.

Comme esters de glycéryle et d'acide gras en C₈-C₂₄ oxyalkylénés, on peut citer par exemple le stéarate de glycéryle (mono-, di- et/ou tri-stéarate de glycéryle) polyoxyéthylèné comme le PEG-20 glyceryl stéarate.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stearate), le monostéarate de polyéthylène glycol 100 OE (nom CTFA : PEG-100 stearate et leurs mélanges.

On peut aussi utiliser des mélanges de ces tensioactifs, comme par exemple le produit contenant du Glyceryl stearate et du PEG-100 stearate, commercialisé sous la dénomination ARLACEL 165 par la société Uniqema, et le produit contenant du Glyceryl stearate (mono-distéarate de glycéryle) et du stéarate de potassium, commercialisé sous la dénomination TEGIN par la société Goldschmidt (nom CTFA : glyceryl stearate SE).

Les esters de sorbitol et d'acide gras en C₈-C₂₄, et leurs dérivés oxyalkylénés peuvent être choisis parmi le palmitate de sorbitan, le trioléate de sorbitan, les esters d'acides gras et de sorbitan oxyalkylénés comprenant par exemple de 20 à 100 OE, comme par exemple le polyethylene sorbitan trioleate (polysorbate 85) ou les composés commercialisés sous les dénominations commerciales Tween 20 ou Tween 60 par la société Ubiqema ;

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dioleate/hydroxystearate) ; l'ester de méthylglucoside et d'acide isostéarique (nom CTFA : Methyl glucose isostearate) ; l'ester de méthylglucoside et d'acide laurique (nom CTFA : Methyl glucose laurate) ; le mélange de monoester et de diester de méthylglucoside et d'acide isostéarique (nom CTFA : Methyl glucose sesqui-isostearate) ; le mélange de monoester et de diester de méthylglucoside et d'acide stéarique (nom CTFA : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Comme éthers de sucre, on peut citer notamment les alkylpolyglucosides, et par exemple le decylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic ; et leurs mélanges.

On peut également citer les mélanges de glycérides d'huiles végétales oxyalkylénés tels que le mélanges glycérides de palme oxyéthyléné (200 OE) et de coprah oxyéthyléné (7 OE).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

Selon un mode de réalisation, la composition A comprend au moins un tensioactif choisi parmi les esters de sucrose, les esters d'acides gras et de sorbitan oxyalkylénés comprenant par exemple de 20 à 100 OE, les esters d'acide gras et de glucose ou d'alkylglucose, comme les sesquistéarates d'alkylglucose, les éthers oxyéthylénés d'acide gras et de glucose ou d'alkylglucose comme les éthers oxyéthylénés d'acide gras et de méthylglucose, les mélanges de glycérides d'huiles végétales oxyalkylénés tels que le mélanges glycérides de palme oxyéthyléné (200 OE) et de coprah oxyéthyléné (7 OE), et leurs mélanges.

### Stucturant lipophile

Selon un mode de réalisation, la composition A comprend avantageusement au moins un agent structurant lipophile (composé distinct de l'agent gélifiant).
L'agent structurant lipophile est choisi parmi les agents structurants polymériques.
Par polymère, on entend au sens de l'invention un composé ayant au moins 2 motifs de répétition, de préférence au moins 3 motifs de répétition et mieux encore 10 motifs de répétition.

La quantité (en matière active) de structurant lipophile peut aller de 0,5% à 20% en poids, de préférence de 1% à 15% en poids et encore mieux de 2% à 10% en poids par rapport au poids total de la composition A.

Les agents structurants polymériques peuvent être notamment choisis parmi a) les polymères de polyamide, b) les copolymères d'oléfine, c) les polymères semi-cristallins, d) les esters de dextrine et d'acide gras, e) les silices pyrogénées, f) les argile organophiles et leurs mélanges.

### a) Polyamides

Les polyamides particuliers utilisés dans la composition selon la présente invention sont de préférence ceux décrits dans le document US-A-5,783,657 de la Société UNION CAMP. La partie de US-A-5,783,657 consacrée à ces polymères est incorporée par référence.

Chacun de ces polyamides satisfait notamment à la formule suivante : 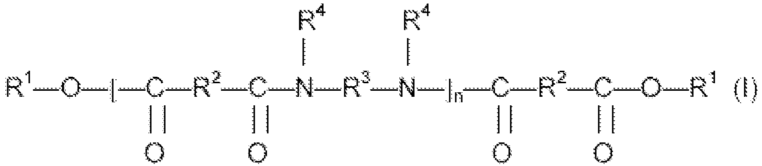 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone et notamment de 4 à 24 atomes de carbone ; R² représente à chaque occurrence indépendamment un groupe hydrocarboné en C₄ à C₅₅ à condition que 50 % au moins des groupes R² représentent un groupe hydrocarboné en C₃₀ à C₅₅ ; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène. En particulier, les groupes ester de ce polyamide représentent de 15 à 40 % du nombre total des groupes ester et amide et au mieux de 20 à 35 %. De plus, n représente avantageusement un nombre entier allant de 1 à 10, et mieux de 1 à 5, bornes incluses.

De préférence, R¹ est un groupe alkyle en C₁₂ à C₂₂ et de préférence en C₁₆ à C₂₂. Avantageusement, R² peut être un groupe hydrocarboné (alkylène) en C₁₀ à C₄₂. De préférence, 50 % au moins et mieux 75 % au moins des R² sont des groupes ayant de 30 à 42 atomes de carbone. Les autres R² sont des groupes hydrogénés en C₄ à C₁₉ et de préférence en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂. Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non. En général, ces polyamides se présentent sous forme de mélanges, ces mélanges pouvant en outre contenir un produit de synthèse correspondant à un polyamide tel que défini ci-dessus avec n valant 0, c'est-à-dire un diester.

Comme polyamide structurant utilisable dans l'invention, on peut encore citer les résines polyamides résultant de la condensation d'un acide di-carboxylique aliphatique et d'un diamine (incluant les composés ayant plus de deux groupes carbonyle et deux groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp., sous la marque Onamid® notamment Onamid S ou C. Ces résines ont une masse moléculaire moyenne en poids allant de 6000 et 9000. Pour plus d'information sur ces polyamides, on peut se référer aux documents US-A-3,645,705 et US-A-3,148,125. Plus spécialement, on utilise les Versamid® 30 ou 744.

On peut aussi utiliser les polyamides vendus ou fabriqués par la société Arizona sous les références Uni-Rez (2658, 2931, 2970, 2621,2613, 2624, 2665, 1554, 2623, 2662) et le produit vendu sous la référence Macromelt 6212 par la société Henkel. Pour plus d'information sur ces polyamides, on peut se référer au document USA-5500209.

A titre d'exemple de polyamides structurant utilisables dans la composition selon l'invention, on peut encore citer les produits commerciaux vendus ou fabriqués par la société Arizona Chemical sous les noms Uniclear 80 et Uniclear 100 VG. Ils sont vendus respectivement sous forme de gel à 80 % (en matière active) et à 100% (en matière active) dans une huile minérale. Ils ont un point de ramollissement de 88 à 105 °C. Ces produits commerciaux sont un mélange de copolymère d'un diacide en C36 condensé sur l'éthylène diamine, de masse moléculaire moyenne d'environ 6000. Les groupes ester terminaux résultent de l'estérification des terminaisons d'acide restantes par l'alcool cétylique, stéarylique ou leurs mélanges (appelés aussi alcool cétylstéarylique).

### b) Copolymère d'oléfine

Par copolymère d'oléfine au sens de la présente demande, on entend tout copolymère formé par polymérisation d'au moins une oléfine et d'un autre monomère additionnel différent de ladite oléfine.

L'oléfine peut être notamment un monomère à insaturation éthylénique.
Comme exemple d'oléfine, on peut citer les monomères de carbure éthylénique, ayant notamment une ou deux insaturation éthylénique, ayant de 2 à 5 atomes de carbone tels que l'éthylène, le propylène, le butadiène, l'isoprène.
Le copolymère d'oléfine choisi de préférence parmi les copolymères d'oléfine amorphes. Par polymère amorphe, on entend un polymère qui n'a pas de forme cristalline. Ce polymère peut être également filmogène, c'est-à-dire qu'il est capable de former un film lors de son application sur la peau.

Le copolymère d'oléfine peut être notamment un copolymère dibloc, tribloc, multibloc, radial, étoile, ou leurs mélanges.
De tels composés sont décrits par exemple dans la demande US-A-2002/005562 et dans le brevet US-A-5 221 534
On choisit avantageusement un copolymère bloc amorphe de styrène et d'oléfine. Le copolymère bloc est de préférence hydrogéné pour réduire les insaturations éthyléniques résiduelles après la polymérisation des monomères.
En particulier, le copolymère bloc hydrocarboné est un copolymère, éventuellement hydrogéné, à blocs styrène et à blocs éthylène/alkylène en C₃-C₄.
Comme copolymère dibloc, de préférence hydrogéné, on peut citer les copolymères de styrène-éthylène/propylène, les copolymères de styrène-éthylène/butadiène, les copolymères de styrène-éthylène/butylène. Des polymères diblocs sont notamment vendus sous la dénomination Kraton® G1701E par la société Kraton Polymers.
Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène.

Des polymères triblocs sont notamment vendus sous les dénominations Kraton® G1650E, Kraton® G1652, Kraton® D1101, Kraton® D1102, Kraton® D1160 par la société Kraton Polymers.
On peut notamment utiliser un copolymère tribloc styrène-éthylène/butylène- styrène.

Selon un mode de réalisation de l'invention, on peut notamment utiliser un mélange d'un copolymère tribloc styrène-butylène/éthylène-styrène et d'un copolymère dibloc styrène-éthylène/butylène, notamment vendus sous la dénomination Kraton® G1657M par la société Kraton Polymers.
On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

### c) Polymères semi-cristallins

Les polymères semi-cristallins sont de préférence des dérivés d'acide acrylique ou méthacrylique. Par "polymère semi-cristallin", on entend au sens de l'invention, des polymères comportant une partie cristallisable, chaîne pendante ou séquence dans le squelette, et une partie amorphe dans le squelette, et présentant une température de changement de phase réversible du premier ordre, en particulier de fusion (transition solide-liquide). Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc.

De façon avantageuse, le ou les polymères semi-cristallins de la composition de l'invention ont une masse moléculaire moyenne en nombre M̅n supérieure ou égale à 2000, allant par exemple de 2 000 à 800 000, de préférence de 3 000 à 500 000, par exemple de 4 000 à 150 000, et mieux de 4 000 à 99 000.

Dans la composition selon l'invention, les polymères semi-cristallins sont avantageusement solubles dans la phase huileuse à au moins 1 % en poids, à une température supérieure à leur température de fusion. En dehors des chaînes ou séquences cristallisables, les séquences des polymères sont amorphes. Par "chaîne ou séquence cristallisable", on entend au sens de l'invention une chaîne ou séquence qui, si elle était seule, passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère.

De préférence, le squelette polymérique des polymères semi-cristallins est soluble dans la phase huileuse.

De façon préférée, les polymères semi-cristallins utilisés dans la composition de l'invention présentent une température de fusion (ou point de fusion), pF, inférieure à 70°C (25°C ≤pF< 70°C), cette température étant au moins égale à la température de la matière kératinique devant recevoir la composition selon l'invention, notamment la peau. La température de fusion peut être mesurée notamment par toute méthode connue et en particulier avec un calorimètre à balayage différentiel (D.S.C).

De préférence, les séquences ou chaînes cristallisables des polymères semi-cristallins représentent au moins 30 % du poids total de chaque polymère et mieux au moins 40 %. Les polymères semi-cristallins à séquences cristallisables utilisés selon l'invention sont des polymères séquencés ou multiséquencés. Ils peuvent être obtenus par polymérisation de monomères à doubles liaisons réactives (ou éthyléniques) ou par polycondensation. Lorsque les polymères de l'invention sont des polymères à chaînes latérales cristallisables, ils sont avantageusement sous forme aléatoire ou statistique.

Les polymères semi-cristallins de l'invention sont d'origine synthétique. En outre, ils ne comportent pas de squelette polysaccharidique.

Les polymères semi-cristallins utilisables dans l'invention sont de préférence choisis parmi les polymères (homopolymères ou copolymères) portant au moins une chaîne latérale cristallisable, et les polymères (homopolymères ou copolymères) portant dans le squelette au moins une séquence cristallisable, comme ceux décrits dans le document US-A-5,156,911. La ou les chaînes latérales ou séquences cristallisables sont hydrophobes.

Selon un mode préféré de réalisation de l'invention, les polymères semi-cristallins sont choisis notamment parmi les homopolymères et copolymères résultant de la polymérisation d'au moins un monomère à chaîne(s) cristallisable(s), celle-ci étant choisie parmi les chaînes alkyles comportant au moins 11 atomes de carbone et au plus 40 atomes de carbone et mieux au plus 24 atomes de carbone. Il s'agit notamment de chaînes alkyle comportant au moins 12 atomes de carbone, et de préférence, il s'agit de chaînes alkyle comportant de 14 à 24 atomes de carbone (C₁₄-C₂₄). Il peut s'agir de chaînes alkyle hydrocarbonées (atomes de carbone et d'hydrogène) ou chaînes alkyle fluorées ou perfluorées (atomes de carbone, atomes de fluor et éventuellement atomes d'hydrogène). Lorsqu'il s'agit de chaînes alkyle fluorées ou perfluorées, elles comportent au moins 11 atomes de carbone dont au moins 6 atomes de carbone sont fluorés.

Par "alkyle", on entend au sens de l'invention un groupement saturé (ne comportant pas d'insaturation).

Selon un mode particulier de réalisation de l'invention, le polymère semi-cristallin est choisi parmi les homopolymères obtenus par polymérisation d'au moins un monomère à chaîne cristallisable, choisi parmi les (méth)acrylates d'alkyle en C₁₄-C₂₄, les (méth)acrylates de perfluoroalkyle en C₁₁-C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle en C₁₄ à C₂₄, et parmi les copolymères de ces monomères, obtenus par copolymérisation de ces monomères avec un monomère hydrophile, de préférence différent de l'acide méthacrylique, comme par exemple la N-vinylpyrrolidone, l'hydroxyéthylacrylate, l'hydroxyéthylméthacrylate, l'acide acrylique. De tels copolymères peuvent être par exemple les copolymères d'alkyl-C₁₄-C₂₄-acrylate, d'alkyl-C₁₄-C₂₄-méthacrylate, d'alkyl-C₁₄-C₂₄-acrylamide, d'alkyl-C₁₄-C₂₄-méthacrylamide, avec la N-vinylpyrrolidone, l'hydroxyéthylacrylate, l'hydroxyéthyl-méthacrylate, l'acide acrylique, ou leurs mélanges.

De préférence , le polymère semi-cristallin est choisi parmi les homopolymère obtenus par polymérisation d'un monomère choisi parmi les acrylates d'alkyle en C₁₄-C₂₄ et les méthacrylates d'alkyle en C₁₄-C₂₄ et parmi les copolymères obtenus par copolymérisation d'un monomère choisi parmi les acrylates d'alkyle en C₁₄-C₂₄ et les méthacrylates d'alkyle en C₁₄-C₂₄, avec un monomère hydrophile tel que l'acide acrylique.

Les polymères semi-cristallins de la composition de l'invention peuvent être non réticulés ou réticulés en partie, du moment que le taux de réticulation ne gène pas leur dissolution ou dispersion dans la phase huileuse par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins de la composition selon l'invention sont non réticulés.

Selon un mode particulier de réalisation de l'invention, le polymère semi-cristallin est un homopolymère résultant de la polymérisation d'un monomère à chaîne cristallisable choisi parmi les acrylates d'alkyle en C₁₄-C₂₄ et les méthacrylates d'alkyle en C₁₄-C₂₄. On peut citer notamment ceux commercialisés sous les dénominations Intelimer® par la société Landec, décrits dans la brochure "Intelimer® polymers", Landec IP22. Ces polymères sont sous forme solide à température ambiante. Ils portent des chaînes latérales cristallisables et correspondent à des homopolymères d'acrylates ou méthacrylates d'alkyle en C₁₄-C₂₄ saturé. On peut citer plus particulièrement l'homopolymère d'acrylate de stéaryle (Intelimer IPA-13.1) (nom INCl : Poly C10-30 alkyl acrylate), l'homopolymère d'acrylate de béhényle (Intelimer IPA-13.6) (nom INCl : Poly C10-30 alkyl acrylate).

Selon un autre mode particulier de réalisation de l'invention, le polymère semi-cristallin est un copolymère d'acrylates d'alkyle en C₁₄-C₂₄ ou de méthacrylates d'alkyle en C₁₄-C₂₄ avec l'acide acrylique. Comme copolymères de ce type, on peut citer les copolymères obtenus par la copolymérisation de l'acrylate de béhényle et de l'acide acrylique, et les copolymères obtenus par la copolymérisation de l'acrylate de stéaryle et de l'acide acrylique.

Selon un mode préféré de réalisation de l'invention, le polymère semi-cristallin est un homopolymère, et il est choisi parmi l'homopolymère d'acrylate de stéaryle (Intelimer IPA-13.1) (nom INCl : Poly C10-30 alkyl acrylate), l'homopolymère d'acrylate de béhényle (Intelimer IPA-13.6) (nom INCl : Poly C10-30 alkyl acrylate), et leurs mélanges.

### d) Ester de dextrine et d'acide(s) gras

La composition selon l'invention comprend au moins un ester de dextrine et d'acide(s) gras.

Plus particulièrement, il s'agit d'un mono-ou poly-ester de dextrine et d'au moins un acide gras et notamment répondant à la formule (C) : 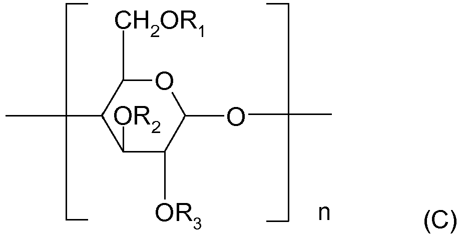 dans laquelle :
- n est un entier allant de 3 à 200, notamment allant de 20 à 150, et en particulier allant de 25 à 50,
- les radicaux R₁, R₂ et R₃, identiques ou différents, sont choisis parmi l'hydrogène ou un groupement acyle (R-CO-) dans lequel le radical R est un groupement hydrocarboné, linéaire ou ramifié, saturé ou insaturé, possédant de 5 à 29, en particulier de 7 à 21, notamment de 11 à 19, plus particulièrement de 13 à 17, voire 15, atomes de carbone, sous réserve qu'au moins un desdits radicaux R₁, R₂ ou R₃ est différent de l'hydrogène.

En particulier, R₁, R₂ et R₃ peuvent représenter l'hydrogène ou un groupement acyle (R-CO-) dans lequel R est un radical hydrocarboné tel que défini précédemment, sous réserve qu'au moins deux desdits radicaux R₁, R₂ ou R₃ sont identiques et différents de l'hydrogène.

L'ensemble des radicaux R₁, R₂ et R₃ peuvent figurer un groupement acyle (R-CO) identique ou différent et notamment identique.

En particulier, n est avantageusement varie de 25 à 50, notamment est égal à 38 dans la formule générale (C) de l'ester selon l'invention.

Notamment lorsque les radicaux R₁, R₂ et/ou R₃, identiques ou différents figurent un groupement acyle (R-CO), ceux-ci peuvent être choisis parmi les radicaux caprylique, caprique, laurique, myristique, palmitique, stéarique, arachique, béhénique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, décénoïque, dodécenoïque, tetradécénoïque, myristoléïque, hexadécénoïque, palmitoléïque, oléïque, élaidique, asclépinique, gondoléïque, eicosènoïque, sorbique, linoléïque, linolénique, punicique, stéaridonique, arachidonique, stéarolique, eicosanyle, docosanoyle, et leurs mélanges.

De préférence, on utilise à titre d'ester de dextrine et d'acide(s) gras au moins un palmitate de dextrine. Celui-ci peut être utilisé seul ou en mélange avec d'autres esters. Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000

Des esters de dextrine, en particulier des palmitates de dextrine, sont disponibles commercialement sous la dénomination RHEOPEARL TL ou RHEOPEARL KL de la société Chiba Flour.

### e) Silices pyrogénées

Les silices pyrogénées sont obtenues par pyrolyse en flamme continue à 1000°C du tétrachlorure de silicium (SiCl₄) en présence d'hydrogène et d'oxygène. Elle peuvent être hydrophiles ou hydrophobes.

On entend par « silice hydrophile » dans la présente demande aussi bien les silices hydrophiles pures que les particules enrobées de silice hydrophile.
On entend par « silice hydrophobe » dans la présente demande aussi bien les silices hydrophobes pures que les particules enrobées de silice hydrophobe.

Parmi les silices pyrogénées à caractère hydrophile utilisables selon la présente invention, on peut citer notamment celles vendues par la société DEGUSSA HÜLS sous les dénominations commerciales AEROSIL 90, 130, 150, 200, 300 et 380.

Parmi les silices pyrogénées à caractère hydrophobe utilisables selon la présente invention, on peut citer notamment celles vendues par la société DEGUSSA HÜLS sous les dénominations commerciales AEROSIL® R202, R805, R812, R972 et R974.

### f) Argiles organophiles

Comme argiles organophiles, on peut citer les argiles modifiées comme les hectorites modifiées par un chlorure d'ammonium en C₁₀ à C₂₂, telle que l'hectorite modifiée par du chlorure de di-stéaryl di-méthyl ammonium telle que, par exemple, celle commercialisée sous la dénomination de Bentone 38V® par la société ELEMENTIS.

L'agent structurant est de préférence choisi parmi les esters de dextrine et d'acide(s) gras, en particulier le palmitate de dextrine.

### Polyols

La composition A comprend avantageusement un polyol qui peut être choisis parmi le glycérol, le sorbitol, le propylène glycol, le pentylène glycol, le butylène glycol, le propanediol , les produits d'addition d'oxyde d'éthylène et d'oxyde d'alkylène en C3-C4 sur un polyol tel que le glycérol comme par exemple le polyoxybutylene polyoxyethylene polyoxypropylene glycerol (nom INCl PEG/PPG/polybutylene glycol-8/5/3 glycerin) tel que le composé commercialisé sous la dénomination WILBRIDE S-753 par NOF, le 2-ethylhexyle ester de glycérol (nom INCl ethyl hexyl glycerine), le caprylyl glycol et leurs mélanges.

Les polyols peuvent représenter de 5% à 40 % en poids, mieux de 10% à 30 % en poids et encore mieux de 12% à 25% en poids de la composition A.

### B. Composition aqueuse

On entend par "composition aqueuse" dans la présente demande, une composition comprenant au moins de l'eau.

La composition aqueuse, outre l'eau, peut contenir un solvant organique soluble dans l'eau, choisi par exemple parmi les mono-alcools inférieurs comportant de 1 à 8 atomes de carbone et en particulier 1 à 6 atomes de carbone, comme l'éthanol, l'isopropanol, le propanol, le butanol ; les polyéthylène glycols ayant de 6 à 80 groupes oxydes d'éthylène ; les polyols comme le propylène glycol, l'isoprène glycol, le butylène glycol, la glycérine, le sorbitol ; l'acétone ; et leurs mélanges.

De préférence, la composition B n'est pas une émulsion.
De préférence, la composition B est une solution aqueuse. Selon un mode de réalisation, elle comprend exclusivement de l'eau et éventuellement au moins un solvant organique hydrosoluble, c'est-à-dire que l'eau et le(s) solvants organiques hydrosolubles éventuellement présents représentent 100% de la composition B. En particulier elle comprend exclusivement de l'eau et un solvant hydrosoluble de l'eau.
De préférence encore, la composition B est exempte de corps gras, notamment d'huiles. L'eau utilisée dans la composition de l'invention peut être de l'eau pure déminéralisée mais aussi de l'eau minérale et/ou de l'eau thermale et/ou de l'eau de mer, c'est-à-dire que l'eau de la composition peut être en partie ou totalement constituée par une eau choisie parmi les eaux minérales, les eaux thermales, les eaux de mer et leurs mélanges. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou contenant des oligo-éléments préparées à partir d'eau purifiée (déminéralisée ou distillée).
Une eau thermale ou minérale naturelle utilisée selon l'invention peut, par exemple, être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

Selon un mode de réalisation, la composition B comprend de préférence, au moins 70%, de préférence au moins 75% et mieux au moins 80% d'eau.

### C/ ADDITIFS

La composition A et/ou la composition B, de préférence la composition A, peut comprendre au moins un additif qui peut être choisi notamment parmi les actifs cosmétiques tels que les actifs hydratants, les agents anti-séborrhéiques, les actifs anti-vieillissement, les actifs anti-rides, les actifs antimicrobiens, les actifs anti-inflammatoires ou apaisants, les actifs lipolytiques ou amincissants ; les charges, les filtres solaires, les agents de coloration de la peau ou des cheveux, les actifs anti-cernes, les actifs anti-transpirants, les actifs déodorants, les actifs des traitements capillaires, les agents dépilatoires, les pigments, les colorants, les parfums, les électrolytes, les ajusteurs de pH, les conservateurs, et leurs mélanges.
I) les actifs hydratants, tels que par exemple le lactate de sodium ; les polyols, et en particulier la glycérine, le sorbitol, les polyéthylène glycols ; le mannitol ; les acides aminés ; l'acide hyaluronique et ses dérivés; la lanoline ; l'urée et les mélanges contenant de l'urée, tels que le NMF (« Natural Moisturising Factor ») ; la vaseline ; l'acide N-lauroyl pyrrolidone carboxylique et ses sels ; les acides gras essentiels ; les huiles essentielles ; et leurs mélanges.
II) les agent anti-séborrhéiques, choisis par exemple parmi :
   - le soufre et les dérivés soufrés ;
   - les sels de zinc tels que le lactate, le gluconate, le pidolate, le carboxylate, le salicylate et/ou le cystéate de zinc ;
   - le chlorure de sélénium ;
   - la vitamine B6 ou pyridoxine ;
   - le mélange de capryloyl glycine, de sarcosine et d'extrait de cinnamomum zeylanicum commercialisé notamment par la société SEPPIC sous la dénomination commerciale Sepicontrol A5® ;
   - un extrait de Laminaria saccharina commercialisé notamment par la société SECMA sous la dénomination commerciale Phlorogine® ;
   - un extrait de Spiraea ulmaria commercialisé notamment par la société SILAB sous la dénomination commerciale Sebonormine® ;
   - des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
   - un extrait de Serenoa repens commercialisé notamment par la société EUROMED ;
   - des extraits de plantes du genre Silybum ;
   - des extraits végétaux contenant des sapogénines et en particulier les extraits de Dioscorées riches en diosgénine ou hécogénine ;
   - des extraits d'Eugenia caryophyllata contenant de l'eugénol et du glucoside d'eugényle ;
   - et leurs mélanges.
III) les actifs anti-vieillissement ou anti-rides peuvent être choisis parmi tous les actifs susceptibles de traiter ou de prévenir tout signe de vieillissement de la peau. Ils peuvent être choisis par exemple parmi les agents anti-radicaux libres, les agents kératolytiques, les vitamines, les agents anti-élastase et anti-collagénase, les protides, les dérivés d'acides gras, les stéroïdes, les oligo-éléments, les agents blanchissants, les extraits d'algues et de planctons, les enzymes et co-enzymes, les flavonoïdes, les céramides, les agents tenseurs, les agents myorelaxants, les sucres, et leurs mélanges.
   1) Comme agents anti-radicaux libres et anti-oxydants, on peut citer notamment les dérivés de l'acide phosphonique tels que l'acide éthylène diamine tétra(méthylène phosphonique), l'acide hexaméthylène diamine tétra(méthylène phosphonique), l'acide diéthylène triamine penta(méthylènephosphonique), et leurs sels et en particulier leurs sels de sodium ; l'acide éthylène diamine tétracétique et ses sels tels que le sel de sodium ; la guanosine ; la superoxydismutase ; le tocophérol (vitamine E) et ses dérivés (acétate) ; l'éthoxyquine ; la lactoferrine ; la lactoperoxydase, et les dérivés nitroxydes ; les superoxyde dismutases ; le glutathion peroxydase ; les extraits végétaux à activité antiradicalaire tels que l'extrait aqueux de germe de blé commercialisé par la société Silab sous la référence Detoxiline ; les extrait végétaux riches en polyphénols tels que le thé vert, le raisin ; et leurs mélanges.
   2) Comme agents kératolytiques, on peut citer par exemple les α-hydroxy-acides notamment les acides dérivés de fruits, comme les acides glycolique, lactique, malique, citrique, tartrique, mandélique, leurs dérivés et leurs mélanges ; les β-hydroxy-acides comme l'acide salicylique et ses dérivés tels que l'acide n-octanoyl-5-salicylique ou l'acide n-dodécanoyl-5-salicylique ; les α-céto-acides comme l'acide ascorbique ou vitamine C et ses dérivés tels que ses sels comme l'ascorbate de sodium, l'ascorbylphosphate de magnésium ou de sodium ; ses esters comme l'acétate d'ascorbyle, le palmitate d'ascorbyle et le propionate d'ascorbyle, ou ses sucres comme l'acide ascorbique glycosylé, et leurs mélanges ; les β-céto-acides ; les rétinoïdes comme le rétinol (vitamine A) et ses esters, le rétinal, l'acide rétinoïque et ses dérivés, ainsi que les rétinoïdes décrits dans les documents FR-A-2,570,377, EP-A-199636, EP-A-325540, EP-A-402072 ; l'adapalène ; les caroténoïdes ; et leurs mélanges.
   3) Comme vitamines, outre les vitamines A, E et C indiquées ci-dessus, on peut citer en particulier la vitamine B3 (ou vitamine PP ou niacinamide) et ses dérivés (nicotinate de tocophérol, esters d'alcool nicotinyle et d'acides carboxyliques, 2-chloronicotinamide, 6-méthylnicotinamide, 6-aminonicotinamide, N-méthyl-nicotinamide, N,N-diméthylnicotinamide, N-(hydroxyméthyl)-nicotinamide, imide d'acide quinolinique, nicotinanilide, N-benzylnicotinamide, N-éthylnicotinamide, nifenazone, nicotinaldéhyde, acide isonicotinique, acide méthylisonicotinique, thionicotinamide, nialamide, acide 2-mercaptonicotinique, nicomol et niaprazine) ; la vitamine B5 (ou panthénol ou alcool panthénylique ou 2,4-dihydroxy-N-(3-hydroxypropyl)-3,3diméthylbutanamide), sous ses différentes formes : D-panthénol, DL-panthénol), et ses dérivés et analogues, tels que le panthoténate de calcium, la panthétine, la pantothéine, l'éther de éthyl panthényl, l'acide pangamique, la pyridoxine, le pantoyl lactose, et les composés naturels en contenant tels que la gelée royale ; la vitamine D et ses analogues tels que ceux décrits dans le document WO-A-00/26167 ; la vitamine F ou ses analogues tels que les mélanges d'acides insaturés possédant au moins une double liaison et notamment les mélanges d'acide linoléique, d'acide linolénique et d'acide arachidonique, ou les composés en contenant et notamment les huiles d'origine végétale en contenant, telles que par exemple l'huile de jojoba, et leurs mélanges.
   4) Comme agents anti-élastase, on peut citer notamment les dérivés peptidiques, et notamment les peptides de graines de légumineuses tels que ceux commercialisés par les Laboratoires Sériobiologiques de Nancy sous la référence Parelastyl ; les dérivés de N-acylamino-amides décrits dans la demande FR-A-2,180,033, comme par exemple le {2-[acétyl-(3-trifluorométhyl-phényl)-amino]-3-méthyl-butyrylamino} acétate d'éthyle et l'acide {2-[acétyl-(3-trifluorométhyl-phényl)-amino]-3-méthyl-butyryl-amino} acétique, et leurs mélanges.
   5) Comme agents anti-collagénase, on peut citer les inhibiteurs de métalloprotéase, tels que l'acide éthylènediamine (EDTA), la cystéine, et leurs mélanges.
   6) Comme protides (ou peptides), on peut citer par exemple les protéines de blé, de riz, de malt ou de soja, leurs hydrolysats, comme ceux commercialisés par la société Silab sous la référence Tensine, et leurs mélanges.
   7) Comme dérivés d'acide gras, on peut citer notamment les phospholipides polyinsaturés dont les phospholipides d'acide gras essentiels desoja, et leurs mélanges.
   8) Comme stéroïdes, on peut citer par exemple la DHEA ou déhydroépi-androstérone, ses précurseurs biologiques, ses métabolites, et leurs mélanges. Par "précurseurs biologiques" de la DHEA, on entend notamment la Δ5-prégnénolone, la 17α-hydroxy prégnénolone et le sulfate de 17α-hydroxy prégnénolone. Par dérivés de la DHEA, on entend aussi bien ses dérivés métaboliques que ses dérivés chimiques. Comme dérivés métaboliques, on peut citer notamment le Δ5-androstène-3,17-diol et notamment le 5-androstène 3β, 17β-diol, la Δ4-androstène-3,17-dione, la 7 hydroxy DHEA (7α-hydroxy DHEA ou 7β-hydroxy-DHEA), la 7-céto-DHEA qui est elle-même un métabolite de la 7β-hydroxy DHEA et la benzoyl DHEA.
   9) Comme oligo-éléments, on peut citer par exemple le cuivre, le zinc, le sélénium, le fer, le magnésium, le manganèse, et leurs mélanges.
   10) Comme agents blanchissants ou dépigmentants, on peut citer par exemple l'acide kojique et ses dérivés ; l'hydroquinone et ses dérivés tels que l'arbutine et ses esters ; la vitamine C et ses dérivés tels que l'ascorbyle phosphate de magnésium ; les sels tels que le calcium D pantéthéine sulfonate ; l'acide ellagique et ses dérivés ; le rucinol ; l'acide linoléique et ses dérivés ; les extraits de plantes, et notamment de réglisse, de mûrier ou de scutellaire ; le glutathion et ses précurseurs ; la cystéine et ses précurseurs ; les composés dérivés d'aminophénol décrits dans le document WO-A-99/10318, comme notamment le N-éthyloxycarbonyl-4-amino-phénol, le N-éthyloxycarbonyl-O-éthyloxycarbonyl-4-amino-phénol, le N-cholestéryloxycarbonyl-4-aminophénol, le N-éthylamino-carbonyl-4-aminophénol ; le phenylethylresorcinol (Symwhite) et les mélanges de ces composés.
   11) Comme extraits d'algues, on peut citer les extraits d'algues rouges ou brunes, et par exemple l'extrait d'algues brunes de la famille des Laminaires, comme les extraits de l'espèce *Laminaria digitata,* et plus particulièrement celui vendu par la société CODIF sous la dénomination Phycosaccharides, qui est une solution concentrée d'un oligosaccharide comprenant l'enchaînement de deux acides uriques : acide mannuronique et l'acide guluronique.
   12) Comme extraits de planctons, on peut citer le plancton en dispersion aqueuse (nom CTFA : Vitreoscilla Ferment) commercialisé sous la dénomination MEXORYL SAH par la société Chimex.
   13) Comme enzymes, on peut utiliser toute enzyme d'origine animale, microbiologique (bactérienne, fongique ou virale) ou synthétique (obtenue par synthèse chimique ou biotechnologique), sous forme cristalline pure ou sous une forme diluée dans un diluant inerte. On peut citer par exemple les lipases, les protéases, les phospholipases, les laccases, les cellulases, les peroxydases notamment les lactoperoxydases, les catalases, les superoxyde dismutases, ou parmi des extraits végétaux contenant les enzymes précitées, et leurs mélanges. Elles peuvent être choisies par exemple parmi celle vendue sous la dénomination commerciale « Subtilisine SP 554 » par la société Novo Nordisk et celle vendue sous la dénomination commerciale « LYSOVEG LS » par la société Laboratoires Sérobiologiques de Nancy.
   14) Comme co-enzymes, on peut utiliser notamment l'ubiquinone ou coenzyme Q10 qui appartient à la famille des benzoquinones à chaîne alkylénée, le coenzyme R qui est la biotine (ou vitamine H), et leurs mélanges.
   15) Comme flavonoïdes, on peut citer par exemple les isoflavonoïdes qui constituent une sous-classe des flavonoïdes, formés d'un squelette 3-phényl chromane qui peut comporter des substituants variés et différents niveaux d'oxydation. Le terme « isoflavonoïde » regroupe plusieurs classes de composés parmi lesquels on peut citer les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromones, les α-méthyldeoxybenzoines, les 2-arylbenzofuranes, et leurs mélanges. Les isoflavonoïdes peuvent être d'origine naturelle ou synthétique. Par "origine naturelle", on entend l'isoflavonoïde à l'état pur ou en solution à différentes concentrations, obtenu par différents procédés d'extraction à partir d'un élément, généralement une plante, d'origine naturelle. Par "origine synthétique", on entend l'isoflavonoïde à l'état pur ou en solution à différentes concentrations, obtenu par synthèse chimique. Comme isoflavonoïdes d'origine naturelle, on peut citer la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites.
   16) Comme céramides, on peut utiliser tout type de céramide, d'origine naturelle ou synthétique, par exemple de type II, de type III, de type IV, de type V ou de type VI, et leurs mélanges. On peut citer par exemple comme céramides, la N-oléoyldihydrosphingosine, la N-stéaroylphytosphingosine, le N-α-hydroxybéhénoyldihydrosphingosine, la N-α-hydroxypalmitoyldihydrosphingosine, la N-linoléoyldihydrosphingosine, la N-palmitoyldihydrosphingosine, la N-stéaroyldihydrosphingosine, la N-béhénoyldihydrosphingosine, et leurs mélanges.
   17) Comme agents tenseurs, on peut citer par exemple :
      - les polymères synthétiques ;
      - les polymères d'origine naturelle ;
      - les silicates mixtes ;
      - les microparticules de cire ;
      - les particules colloïdales de charges inorganiques.
         - Les polymères synthétiques utilisables en tant qu'agent tenseur peuvent être choisis parmi:
            - les polymères et copolymères de polyuréthanne ;
            - les polymères et copolymères acryliques ;
            - les polymères d'acide isophtalique sulfoné ;
            - les polymères siliconés greffés ;
            - les polymères hydrosolubles ou hydrodispersibles comprenant des unités hydrosolubles ou hydrodispersibles et des unités à LCST (Lower Critical Solution Temperature).
         - Les copolymères de polyuréthanne, les copolymères acryliques et les autres polymères synthétiques pouvant être utilisés comme agents tenseurs peuvent notamment être choisis parmi les polycondensats, les polymères hybrides et les réseaux de polymères interpénétrés (IPNs). Par "réseau de polymères interpénétrés", on entend un mélange de deux polymères enchevêtrés, obtenu par polymérisation et/ou réticulation simultanée de deux types de monomères, le mélange obtenu ayant une température de transition vitreuse unique. Des exemples d'IPNs convenant comme polymères tenseurs, ainsi que leur procédé de préparation, sont décrits par exemple dans les documents US-A-6,139,322 et US-A-6,465,001. De préférence, l'IPN comprend au moins un polymère polyacrylique et, plus préférentiellement, il comprend en outre au moins un polyuréthane ou un copolymère de fluorure de vinylidène et d'hexafluoropropylène. Selon une forme d'exécution préférée, l'IPN comprend un polymère polyuréthane et un polymère polyacrylique. De tels IPNs sont notamment ceux de la série Hybridur commercialisés par la société Air Products. Un IPN particulièrement préféré comme polymère tenseur se trouve sous la forme d'une dispersion aqueuse de particules ayant une taille moyenne en poids comprise entre 90 et 110 nm et une taille moyenne en nombre d'environ 80 nm. Cet IPN a de préférence une température de transition vitreuse, Tg, qui va d'environ -60°C à +100°C. Un IPN de ce type est notamment commercialisé par la société Air Products sous la dénomination commerciale Hybridur X-01602. Un autre IPN convenant à une utilisation dans la présente invention est référencé Hybridur X18693-21 ou Hybridur 875 polymer dispersion.
         - D'autres IPNs convenant comme polymères tenseurs comprennent les IPNs constitués du mélange d'un polyuréthane avec un copolymère de fluorure de vinylidène et d'hexafluoropropylène, notamment ceux préparés comme décrit dans le document US-A-5,349,003. En variante, ils sont disponibles dans le commerce sous forme de dispersion colloïdale dans l'eau, dans un rapport du copolymère fluoré au polymère acrylique comprise entre 70:30 et 75:25, sous les dénominations commerciales KYNAR RC-10,147 et KYNAR RC-10,151 auprès de la société Atofina.
         - Des exemples de polymères siliconés greffés sont indiqués dans le document EP-A-1,038,519, qui est incorporé ici par référence. Un exemple préféré de polymère siliconé greffé est le polysilicone-8 (nom CTFA) qui est un polydiméthylsiloxane sur lequel sont greffés, par l'intermédiaire d'un chaînon de raccordement de type thiopropylène, des motifs polymères mixtes du type acide poly(méth)acrylique et du type poly(méth)acrylate d'alkyle. Un polymère de ce type est notamment disponible sous la dénomination commerciale VS 80 (à 10% dans l'eau) ou LO 21 (sous forme pulvérulente) auprès de la société 3M. Il s'agit d'un copolymère de polydiméthylsiloxane à groupements propylthio, d'acrylate de méthyle, de méthacrylate de méthyle et d'acide méthacrylique.
         - Les polymères synthétiques susmentionnés peuvent se présenter sous forme de latex. Comme latex approprié pouvant être utilisé en tant qu'agent tenseur, on peut citer notamment les dispersions de polyester-polyuréthane et de polyéther-polyuréthane, telles que celles commercialisées sous les dénominations Avalure UR410 et UR460 par la société Noveon, et sous les dénominations Neorez R974, Neorez R981, Neorez R970, ainsi que les dispersions de copolymère acrylique telles que celles commercialisées sous la dénomination Neocryl XK-90 par la société Avecia.
         - Enfin, des polymères synthétiques appropriés comme polymères tenseurs peuvent être des polymères hydrosolubles ou hydrodispersibles comprenant des unités hydrosolubles ou hydrodispersibles et comprenant des unités à LSCT, lesdites unités à LCST présentant, en particulier, une température de démixtion dans l'eau de 5 à 40°C à une concentration massique de 1%. Ce type de polymère est plus amplement décrit dans le document FR-A-2,819,429.
         - Les polymères d'origine naturelle utilisables en tant qu'agent tenseur peuvent être choisis parmi :
            - les protéines végétales et hydrolysats de protéines végétales ;
            - les polysaccharides d'origine végétale, éventuellement sous forme de microgels, tels que l'amidon ;
            - les latex d'origine végétale.
         - Comme exemples de protéines végétales et hydrolysats de protéines végétales utilisables comme agents tenseurs, on peut citer les protéines et hydrolysats de protéines de maïs, de seigle, de froment, de sarrasin, de sésame, d'épeautre, de pois, de fève, de lentille, de soja et de lupin.
         - Les polysaccharides utilisables comme agents tenseurs peuvent être choisis parmi les polysaccharides d'origine naturelle, capables de former des gels thermoréversibles ou réticulés ainsi que des solutions. On entend par "thermoréversible" le fait que l'état gel de ces solutions de polymère est obtenu de façon réversible, une fois la solution refroidie en-dessous de la température de gélification caractéristique du polysaccharide utilisé. Comme polysaccharides d'origine naturelle de ce type, on peut citer les carraghénanes et tout particulièrement le kappa-carraghénane et le iota-carraghénane ; les agars ; les gellanes ; les alginates ; les pectines ; les chitosanes et leurs dérivés ; les pullulanes et leurs dérivés. Ces polysaccharides tenseurs peuvent être présents sous forme de microgels tels que décrits dans le document FR-A-2,829,025.
         - Les polysaccharides peuvent être choisis aussi parmi l'amidon et ses dérivés. L'amidon peut être de toute origine : par exemple riz, maïs, pomme de terre, manioc, pois, froment, avoine, et il peut être naturel ou éventuellement modifié par un traitement de type réticulation, acétylation, oxydation. Il peut être éventuellement greffé. Comme amidon pouvant être utilisé comme agent tenseur, on peut citer par exemple celui commercialisé par la société Lambert-Rivière sous la dénomination Remi Dri.
         - Une autre classe d'agents tenseurs utilisables selon l'invention est constituée par les silicates mixtes. Par cette expression, on entend tous les silicates d'origine naturelle ou synthétique renfermant plusieurs types de cations choisis parmi les métaux alcalins (par exemple Na, Li, K) ou alcalino-terreux (par exemple Be, Mg, Ca) et les métaux de transition. On utilise de préférence des phyllosilicates, à savoir des silicates ayant une structure dans laquelle les tétraèdres SiO₄ sont organisés en feuillets entre lesquels se trouvent enfermés les cations métalliques. Une famille de silicates particulièrement préférée comme agents tenseurs est celle des laponites. Les laponites sont des silicates de magnésium, de lithium et de sodium ayant une structure en couches semblable à celle des montmorillonites. La laponite est la forme synthétique du minéral naturel appelé "hectorite". On peut utiliser par exemple la laponite commercialisée sous la dénomination Laponite XLS ou Laponite XLG par la société Rockwood.
         - Une autre classe encore d'agents tenseurs est constituée par les microparticules de cire. Il s'agit de particules ayant un diamètre généralement inférieur à 5 µm, ou mieux à 0,5 µm, et constituées essentiellement d'une cire ou d'un mélange de cires choisies par exemple parmi les cires de Carnauba, de Candelilla ou d'Alfa. Le point de fusion de la cire ou du mélange de cires est de préférence compris entre 50°C et 150°C.
         - En variante encore, on peut utiliser comme agent tenseur, des particules colloïdales de charges inorganiques. Par "particules colloïdales", on entend des particules colloïdales en dispersion dans un milieu aqueux, hydroalcoolique ou alcoolique, ayant un diamètre moyen en nombre compris entre 0,1 et 100 nm, de préférence entre 3 et 30 nm. Comme exemples de charges inorganiques, on peut citer la silice, l'oxyde de cérium, l'oxyde de zirconium, l'alumine, le carbonate de calcium, le sulfate de baryum, le sulfate de calcium, l'oxyde de zinc et le dioxyde de titane. Une charge inorganique particulièrement préférée est la silice. Des particules colloïdales de silice sont notamment disponibles sous forme de dispersion aqueuse de silice colloïdale auprès de la société catalysts & Chemicals sous les dénominations commerciales COSMO S-40 et COSMO S-50. on peut aussi utiliser des particules colloïdales composites silice-alumine, telles que celles commercialisées par la société Grace sous les noms de Ludox AM, Ludox HSA et Ludox TMA.
   18) Comme agents myorelaxants qui sont des agents de lissage des rides d'expression, on peut citer par exemple les sapogénines (voir demande EP-A-1,352,643) ; l'adénosine (voir demande FR-0214828); les antagonistes des récepteurs associés aux canaux calciques (voir demande FR-A-2,793,681), et en particulier le manganèse et ses sels (voir demande FR-A-2,809,005) et l'alvérine (voir demande FR-A-2,798,590) ; les agonistes des récepteurs associés aux canaux chlore, dont la glycine (voir demande EP-A-0704210) et certains extraits d'*Iris pallida* (voir demande FR-A-2,746,641).
   19) Comme sucres, on peut citer les monosaccharides comme le D-mannose, le L-rhamnose, les polysaccharides, les C-glycosides et leurs dérivés, tels que le C-β-D-xylopyranoside-n-propane-2-one, le C-β-D-(3.4.5-triacetoxy)xylopyranoside-n-propane-2-one, le C-β-D-xylopyranoside-2-hydroxy-propane-2-one et leurs mélanges.
IV) les actifs antimicrobiens ou antifongiques et les conservateurs, notamment l'hexamidine iséthionate, l'acide undécylenique et ses sels, le peroxyde de benzoyle, l'acide benzoïque et ses sels, l'acide phytique et ses sels, l'acide salicylique et ses sels, l'acide sorbique et ses sels, l'acide levulinique, l'alcool benzylique, l'acide dehydroacétique et ses sels, l'acide anisique, l'acide N-acétyl-L-cystéine, l'acide lipoïque, l'acide azélaïque et ses sels, l'acide arachidonique, le résorcinol, le 2,4,4'-trichloro-2'-hydroxydiphényl éther, l'octopirox, l'octoxyglycérine, l'octanoylglycine, le capryloyl glycine, le farnesol, les phytosphingosines, les dérivés du sélénium, le pyrithione zinc, la chlophénésine, le phénoxyéthanol, les esters de l'acide parahydroxybenzoïque encore appelés Parabens, les libérateurs de formol comme l'imidazolidinyl urée ou la diazolidinyl urée ; les halogénoalkynylcarbamates comme le 3-iodo-2-propynyl butylcarbamate (IPBC) ; le caprylylglycol encore appelé 1,2-octanediol ; le pentylène glycol ; le chlorure de N-(3-chloroallyl)-hexaminium (ou Quaternium-15) ; le chlorhydrate de polyhexaméthylène biguanide (nom CTFA : polyaminopropyl biguanide) ; les bromures d'alkyl-triméthylammonium comme le bromure de dodécyl-triméthylammonium, le bromure de myristyl-triméthylammonium, le bromure d'hexadécyl- triméthylammonium, et leurs mélanges. Ces conservateurs peuvent être notamment présents dans la composition aqueuse.
V) les actifs anti-inflammatoires ou apaisants tels que les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stéaryl glycyrrhétinate, l'acide 3- stéaroyloxy glycyrrhétique) ; l'acide ursolique et ses sels ; l'acide oléanolique et ses sels ; l'acide bétulinique et ses sels ; les extraits de Paeonia suffruticosa, de lactiflora, de Laminaria saccharina, de camomille, du Pygeum, de Boswellia serrata, de Centipeda cunnighami, d'Helianthus annuus, de Linum usitatissimum, de Cola nitida, de clou de girofle, d'Epilobium Angustifolium, de Bacopa monieri ; les sels de l'acide salicylique et en particulier le salicylate de zinc ; les phycosaccharides de la société Codif ; l'huile de Canola ; le bisabolol ; l'allantoïne ; le Sépivital EPC (diesterphosphorique de vitamine E et C) de la société Seppic ; les huiles insaturées en oméga 3 telles que les huiles de rosier muscat, de cassis, d'Echium, de poisson ; la capryloyl glycine ; le Seppicalm VG (sodium palmitoylproline et nymphea alba) de la société Seppic ; les tocotrienols ; le piperonal, l'aloe vera ; les phytostérols.
VI) les actifs lipolytiques ou amincissants, c'est-à-dire ayant une activité favorable, directe ou indirecte, sur la diminution du tissu adipeux, tels que :
   - les dérivés xanthiques comme la caféine et ses dérivés, notamment les 1-hydroxyalkylxanthines décrites dans le document FR-A-2,617,401, la caféine citrate, la théophylline et ses dérivés, la théobromine, l'acéfylline, l'aminophylline, le chloroéthylthéophylline, le diprofylline, le diniprophylline, l'étamiphylline et ses dérivés, l'étofylline, la proxyphylline ; ou les associations contenant des dérivés xanthiques, comme l'association de caféine et de silanol (dérivé méthylsilanetriol de caféine), et par exemple le produit commercialisé par la société Exsymol sous la dénomination caféisilane C ;ou bien les composés d'origine naturelle contenant des bases xanthiques et notamment de la caféine, tels que les extraits de thé, de café, de guarana, de maté, de cola (*Cola Nitida*) et notamment l'extrait sec de fruit de guarana (*Paulina sorbilis*) contenant 8 à 10 % de caféine ; l'éphédrine et ses dérivés qui peuvent notamment se retrouver à l'état naturel dans les plantes telles que le Ma Huang (Ephedra plant) ;
   - les extraits végétaux et les extraits d'origine marine, qui soit sont actifs sur les récepteurs à inhiber, tels que les β-2-bloqueurs, les NPY-bloqueurs (décrits dans le document EP-A-838217), soit inhibent la synthèse des récepteurs aux LDL ou VLDL, soit sont actifs pour stimuler les récepteurs β et les protéines G, conduisant à l'activation de l'adénylcyclase. Comme extraits végétaux de ce type, on peut citer par exemple :
      - le *Garcinia Cambogia,*
      - les extraits de *Bupleurum chinensis,*
      - les extraits de lierre grimpant (*Hedera Helix*), d'arnica (*Arnica Montana L*), de romarin (*Rosmarinus officinalis N*), de souci (*Calendula officinalis*), de sauge (*Salvia officinalis L*), de ginseng (*Panax ginseng*), de millepertuis (*Byperycum Perforatum*), de fragon (*Ruscus aculeatus L*), d'ulmaire (*Filipendula ulmaria L*), d'orthosiphon (*Orthosiphon Stamincus Benth*), de bouleau (*Betula alba*), de cécropia et d'arganier,
      - les extraits de ginkgo biloba,
      - les extraits de prêle,
      - les extraits d'escine,
      - les extraits de cangzhu,
      - les extraits de *chrysanthellum indicum,*
      - les extraits de dioscorés riches en diosgénine ou la diosgénine ou hécogénine pure et leurs dérivés.
      - les extraits des plantes du genre *Armeniacea, Atractylodis Platicodon, Sinom-menum, Pharbitidis, Flemingia,*
      - les extraits de *Coleus* tels que *C. Forskohlii, C. blumei, C. esquirolii, C. scutellaroïdes, C. xanthantus* et *C. Barbatus,* tel que l'extrait de racine de *Coleus Barbatus* contenant 60 % de forskoline,
      - les extraits de Ballote,
      - les extraits de *Guioa,* de *Davallia,* de *Terminalia,* de *Barringtonia,* de *Trema,* d'*Antirobia.*
   Comme extrait d'origine marine on peut citer :
   - les extraits d'algues ou de phytopancton, tels que le rhodystérol ou l'extrait de *Laminaria Digitata* commercialisé sous la dénomination PHYCOX75 par la société Secma, l'algue skeletonema décrite dans le document FR-A-2,782,921 ou les diatomées décrites dans le document FR-A-2,774,292.
VII) Les charges, et spécialement celles qui apportent des effets optiques, c'est-à-dire les charges capables de conférer après application sur la peau, les effets optiques suivants :
   - matité ou diminution de la brillance ;
   - effet Soft Focus : On entend par effet «soft focus» la diminution visible des ridules, des pores et des irrégularités du micro relief de la peau, diminution obtenue instantanément après application de la composition cosmétique sur une peau présentant des imperfections liées à un micro relief irrégulier ;
   - homogénéisation et éclaircissement du teint : On entend par "homogénéité du teint", la diminution visible des dyschromies et irrégularités pigmentaires de la peau, obtenue instantanément après application de la composition cosmétique sur une peau présentant ces imperfections.

   Les charges sont des particules solides, généralement blanches, insolubles dans le milieu de la composition.
   Parmi les charges à effet optique utilisables dans l'invention, on peut citer les charges minérales (dioxyde de titane amorphe ou cristallisé sous forme rutile et/ou anatase, oxyde de zinc, oxyde de fer, oxyde de cérium, silice, alumine, nitrure de bore, talc, séricite, mica, ...) enrobées ou non ainsi que les charges composites, les nacres, les argiles, l'amidon et ses dérivés, les dispersions aqueuses de styrène acrylique, les particules de résine de mélamine-formaldéhyde ou d'urée-formaldéhyde, les dispersions aqueuses de polytétrafluoroéthylène (PTFE), les microdispersions de cires, les copolymères vinylpyrrolidone/1-triacontène, les cires et résines de silicone, les particules d'organopolysiloxane, les microsphères de terpolymère expansé de chlorure de vinylidène, d'acrylonitrile et de méthacrylate, les particules de nylon, les microbilles de cellulose, les fibres, les particules hémisphériques creuses de silicone tells que celles commercialisée sous les dénominations NLK-500 et NLK-503 par la société Takemoto Oil and Fat.
   On peut également citer les élastomères de silicone (organopolysiloxanes élastomères) de préférence partiellement ou totalement réticulés, se présentant sous forme de particules comme par exemple ceux commercialisés sous les références KSG 15, KSG 16, KSG 17, KSG 18, KSG 26A, KSG 26B, KSG-31", "KSG-32", "KSG-33", "KSG-41", "KSG-42", "KSG-43", "KSG-44" par la société Shin-Etsu ;
VIII) les filtres solaires, qui peuvent être choisis parmi les filtres chimiques UVA et UVB ou les filtres physiques habituellement utilisables dans le domaine cosmétique.
   Comme filtres UVB, on peut citer par exemple :
   (1) les dérivés de l'acide salicylique, en particulier le salicylate d'homomenthyle et le salicylate d'octyle ;
   (2) les dérivés de l'acide cinnamique, en particulier le p-méthoxycinnamate de 2-éthylhexyle, commercialisé par la société Givaudan sous la dénomination Parsol MCX ;
   (3) les dérivés de β,β'-diphénylacrylate liquides, en particulier l'α-cyano-α,β' diphénylacrylate de 2-éthylhexyle ou octocrylène, commercialisé par la société BASF sous la dénomination UVINUL N539 ;
   (4) les dérivés de l'acide p-aminobenzoïque ;
   (5) le 4-méthyl benzylidène camphre commercialisé par la société Merck sous la dénomination EUSOLEX 6300 ;
   (6) l'acide 2-phénylbenzimidazole 5-sulfonique commercialisé sous la dénomination EUSOLEX 232 par la société Merck ;
   (7) les dérivés de 1,3,5-triazine, en particulier :
      - la 2,4,6-tris[p-(2'-éthylhexyl-1'-oxycarbonyl)anilino]-1,3,5-triazine commercialisé par la société BASF sous la dénomination UVINUL T150, et
      - le dioctyl butamido triazone commercialisé par la société Sigma 3V sous la dénomination UVASORB HEB ;
   (8) les mélanges de ces filtres.

   Comme filtres UVA, on peut citer par exemple :
   (1) les dérivés de dibenzoylméthane, en particulier le 4-(tert.-butyl) 4'-méthoxy dibenzoylméthane commercialisé par la société Givaudan sous la dénomination PARSOL 1789 ;
   (2) l'acide benzène 1,4 [di(3-méthylidènecampho-10-sulfonique)] éventuellement sous forme partiellement ou totalement neutralisée, commercialisé sous la dénomination MEXORYL SX par la société Chimex.
   (3) les dérivés de benzophénone, par exemple :
      - la 2,4-dihydroxybenzophénone (benzophénone-1) ;
      - la 2,2',4,4'-tétra-hydroxybenzophénone (benzophénone-2) ;
      - la 2-hydroxy-4-méthoxy-benzophénone (benzophénone-3), commercialisé sous la dénomination UVINUL M40 par la société BASF ;
      - l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40 ;
      - la 2,2'-dihydroxy-4,4'-diméthoxy-benzophénone (benzophénone-6) ;
      - la 5-chloro-2-hydroxybenzophénone (benzophénone-7) ;
      - la 2,2'-dihydroxy-4-méthoxy-benzophénone (benzophénone-8) ;
      - le sel disodique du diacide 2,2'-dihydroxy-4,4'-diméthoxy-benzophénone-5,5'-disulfonique (benzophénone-9) ;
      - la 2-hydroxy-4-méthoxy-4'-méthyl-benzophénone (benzophénone-10) ;
      - la benzophénone-11 ;
      - la 2-hydroxy-4-(octyloxy)benzophénone (benzophénone-12).
   (4) les dérivés silanes ou les polyorganosiloxanes à groupement benzophénone ;
   (5) les anthranilates, en particulier l'anthranilate de menthyle commercialisé par la société Haarman & Reiner sous la dénomination NEO HELIOPAN MA ;
   (6) les composés comportant par molécule au moins deux groupes benzoazolyle ou au moins un groupe benzodiazolyle, en particulier l'acide 1,4-bis-benzimidazolyl-phenylèn-3,3',5,5'-tétrasulfonique ainsi que ses sels commercialisés par la société Haarman & Reimer ;
   (7) les dérivés siliciés de benzimidazolyl-benzazoles N-substitués ou de benzofuranyl-benzazoles, et en particulier :
      - le 2-[1-[3-[1,3,3,3-tétraméthyl-1-[(triméthylsilyl)oxy]disiloxanyl]propyl]-1H-benzimidazol-2-yl]-benzoxazole ;
      - le 2-[1-[3-[1,3,3,3-tétraméthyl-1-[(triméthylsilyl)oxy]disiloxanyl]propyl]-1H-benzimidazol-2-yl]-benzothiazole ;
      - le 2-[1-(3-trimethylsilanyl-propyl)-1H-benzimidazol-2-yl]-benzoxazole ;
      - le 6-méthoxy-1,1'-bis-(3-triméthylsilanyl-propyl)-1H,1'H-[2,2']bibenzimidazolyl-benzoxazole ;
      - le 2-[1-(3-trimethylsilanyl-propyl)-1H-benzimidazol-2-yl]-benzothiazole, qui sont décrits dans le document EP-A-1 028 120 ;
   (8) les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M ;
   (9) Les silicones benzotriazoles, qui sont décrits notamment dans le document EP-A-0392 883, en particulier le silicone benzotriazole de formule : 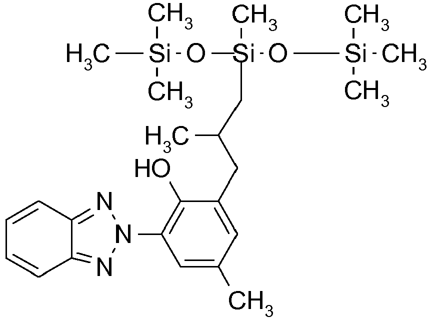
   (10) leurs mélanges.

On peut aussi utiliser un mélange de plusieurs de ces filtres.
IX) les agents de coloration de la peau et des cheveux, en particulier les agents de la coloration de la peau tels que la dihydroxyacétone (DHA), les colorants naturels tels que les extraits végétaux comme par exemple les extraits de sorgho, et les azurants optiques. Les azurants optiques sont une famille de substances fluorescentes bien connues de l'homme du métier. De tels composés sont décrits dans "Fluorescent Whitening Agent, Encyclopedia of Chemical Technology, Kirk-Othmer", vol 11, p. 227-241, 4ème édition, 1994, Wiley. Ce sont des agents de blanchiment de la peau par voie optique, constitués par des composés chimiques dotés de propriétés de fluorescence, qui absorbent dans l'ultraviolet (absorption maximale à une longueur d'onde inférieure à 400 nm) et réémettent de l'énergie par fluorescence pour une longueur d'onde comprise entre 380 nm et 830 nm. Une émission d'énergie comprise entre 400 nm et 480 nm résulte en une émission dans le bleu du domaine visible, ce qui contribue, lorsque cette émission a lieu sur la peau, à la blanchir visuellement.
   On peut plus particulièrement les définir comme des composés qui absorbent essentiellement dans l'UVA et l'UVB entre 290 et 400 nm et réémettent essentiellement entre 400 et 525 nm. Parmi les azurants optiques, on utilise de préférence des dérivés du stilbène, des dérivés coumariniques, des dérivés oxazole et benzoxazole, des dérivés imidazole. De tels composés sont facilement disponibles commercialement. Les azurants optiques préférentiellement utilisés dans le produit selon l'invention sont le di-styryl-4,4'bi-phenyl di-sulfonate commercialisés par la société Ciba Geigy sous le nom de Tinopal CBS-X ®. On peut aussi citer par exemple le 4,4'-bis[(4,6-diamilino-1,3,5-triazin-2-yl)amino]stilbène-2,2'-disulfonate de sodium, et le 2,5 thiophène di-yl bis(5 ter-butyl-1,3 benzoxazole) commercialisés par la société Ciba Geigy sous les noms de Tinopal SOP ® et d'Uvitex OB ®.
X) les actif anti-cernes tels que la vitamine K1 et ses dérivés, et les coumarines.
XI) les actifs anti-transpirants tels que les sels d'aluminium et/ou de zirconium, comme par exemple la perlite, le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tétrachlorohydrate, l'aluminium zirconium trichlorohydrate et plus particulièrement l'aluminium chlorhydrate commercialisé par la société REHEIS sous la dénomination REACH 301 ou 303 ou par la société GUILINI CHEMIE sous la dénomination ALOXICOLL PF 40, et le sel d'aluminium et de zirconium commercialisé par la société REHEIS sous la dénomination REACH AZP-908-SUF ; les complexes d'hydroxychlorure de zirconium et d'hydroxychlorure d'aluminium avec un acide aminé tels que ceux décrits dans le document US-A-3,792,068, communément connus sous l'appellation "ZAG complexes", comme par exemple l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.
XII) Les actifs déodorants, tels que le pyrrolidone carboxylate de zinc (plus communément appelé pidolate de zinc), le sulfate de zinc, le chlorure de zinc, le lactate de zinc, le gluconate de zinc et le phénolsulfonate de zinc, le 2,4,4'-trichloro-2'-hydroxydiphényléther (Triclosan), le 2,4-dichloro-2'-hydroxydiphényléther, le 3',4',5'-trichlorosalicylanilide, le 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol, le monolaurate de glycérol ; les sels de polyhexaméthylène biguanide.
XIII) Les actifs des traitements capillaires, tels que (1) les agents inhibiteurs de la chute du cheveu ainsi que les agents stimulateurs de la pousse du cheveu, tels que le minoxidil, la biotine, l'aminexil, la cystéine, le finastèride, le 2,4 dipirymidine N-oxyde, le panthénol et dérivés, la flavanone T, les antagonistes de calcium comme le diltiazem, le vérapamil, l'alvérine, et la nifédipine, les hormones comme la progestérone, les agonistes du récepteur FP comme le latanoprost, les inhibiteurs de la 15-hydroxy prostaglandine déshydrogénase de type 1, les prostaglandines et leurs dérivés, ou plus généralement tout extrait végétal possédant une activité anti-5-alpha réductase de type I ou II ; (2) les agents anti-pelliculaires tels que la pyrithione de zinc, les dérivés de 1-hydroxy-2-pyrrolidone ou encore les sulfures de sélénium.
XIV) Les agents dépilatoires qui sont utilisés pour inhiber la pousse des poils, tels que l'acide thioglycolique et ses dérivés ; l'acide dithioglycolique et ses dérivés ; les sérines protéases décrites dans le document US-A-6,407,056 ; l'acide caféique ; la quercétine ; le gallate de propyle ; l'acide norhydroguaiarétique ou NDGA ; l'indométhacine ; l'hydrochlorure d'eflornithine ; les extraits végétaux tels que décrits dans le document US-A-6,171,595, comme les extraits de clou de girofle, de bouton de rose, de pimprenelle (burnet), de gambir ; les composés décrits dans le document US-A-6,075,052 ; le tétramisole ; l'orthovanadate de sodium ; le levamisole ; le chromoglycate disodique ; le nitrate de vanadium et le nitrate de gallium tel que décrit dans le document US-A-6,020,006 ; les composés décrits dans les documents US-A-4,885,289, US-A-4,720,489, US-A-5,132,293, US-A-5,096,911, US-A-5,095,007, US-A-5,143,925, US-A-5,328,686, US-A-5,440,090, US-A-5,364,885, US-A-5,411,991, US-A-5,648,394, US-A-5,468,476, US-A-5,475,763, US-A-5,455,608, US-A-5,674,477, US-A-5,728,736 et US-A-5,652,273, WO-A-94/27586, WO-A-94/27563 et WO-A-98/03149 ; les extraits de genévrier tels que décrits dans le document US-A-6,375,948.
XV) Les pigments qui sont utilisés notamment quand le produit obtenu est destiné au maquillage de la peau ou, quand il s'agit de pigment d'oxydes métalliques, quand le produit obtenu est destiné à constituer un produit solaire pour protéger la peau ou les cheveux colorés des rayons du soleil.
   Ces pigments peuvent être minéraux et/ou organiques, interférentiels, goniochromatiques, fluorescents, blancs, colorés, nacrés ou réfléchissants ou sous forme de paillettes. Par pigment, il faut comprendre des particules insolubles dans le milieu physiologique de la composition.
   On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium ou aluminium dont celles soumises à une certification par la Food and Drug Administration (FDA) (exemple D&C ou FD&C) et ceux exempts de la certification FDA comme les laques à base de carmin de cochenille.
   Les pigments nacrés ou nacres peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Parmi les nacres disponibles sur le marché, on peut citer les nacres commercialisées sous les dénominations TIMICA® et FLAMENCO® par la société Engelhard et les nacres commercialisées sous la dénomination TIMIRON® par la société Merck.
   On peut aussi utiliser des pigments goniochromatiques, comme les pigments à structure multicouche interférentielle par exemple de structure Al/SO₂/Al/SiO₂/Al, commercialisés par la société Dupont de Nemours ; de structure Cr/MgF₂/Al/MgF₂/Cr commercialisés sous la dénomination CHROMAFLAIR® par la société Flex ; de structure MoS₂/SiO₂/Al/SiO₂/MoS₂, Fe₂O₃/SiO₂/Al/SiO₂/Fe₂O₃ ou Fe₂O₃/SiO₂/Fe₂O₃/SiO₂/Fe₂O₃ commercialisés sous la dénomination de SICOPEARL® par la société BASF ; de structure MoS₂/SiO₂/mica-oxyde/SiO₂/MoS₂, Fe₂O₃/SiO₂/mica-oxyde/SiO₂/Fe₂O₃, TiO₂/SiO₂/TiO₂ ou TiO₂/Al₂O₃/TiO₂, commercialisés sous la dénomination XIRONA® par la société Merck. On peut encore citer les pigments commercialisés sous la dénomination INFINITE COLORS® de la société Shiseido.
   On peut aussi utiliser des pigments réfléchissants, comme des particules à substrat de verre revêtu d'argent, en forme de plaquettes, telles que celles vendues par exemple sous la dénomination MICROGLASS METASHINE REFSX 2025 PS® par la société Toyal ; des particules à substrat de verre revêtu d'alliage nickel/chrome/molybdène, telles que celles vendues par exemple sous les dénominations CRYSTAL STAR GF 55®, GF 2525® par la société Toyal ; des pigments de marque REFLECKS®, commercialisés par la société Engelhard, comportant un substrat de verre enrobé d'oxyde de fer brun ; des particules comportant un empilement d'au moins deux couches de polymères comme celles commercialisées par la société 3M sous la dénomination MIRROR GLITTER®.
   Comme particules goniochromatiques à cristaux liquides, on peut utiliser par exemple celles vendues par la société Chenix ainsi que celle commercialisée sous la dénomination HELICONE® HC par la société Wacker.
XVI) Les colorants qui vont apporter une couleur au(x) film(s) et/:ou à la composition aqueuse, notamment les colorants hydrosolubles tels que le sulfate de cuivre, le sulfate de fer, les sulfopolyesters hydrosolubles, les rhodamines, les colorants naturels comme le carotène et le jus de betterave, le bleu de méthylène, le caramel, le sel disodique de tartrazine et le sel disodique de fuschine, et leurs mélanges. On peut aussi éventuellement utiliser des colorants liposolubles. Le ou les colorants sont de préférence présents dans le ou les films. La composition aqueuse peut ainsi être colorée au moment de l'utilisation selon la teinte souhaitée à ce moment-là.
   Les colorants et les pigments permettent de moduler la couleur en fonction du but recherché (effet bonne mine, anti-cernes par exemple).
XVII) Les parfums qui peuvent être de tout type, soit des parfums composites comprenant un mélange de matières odoriférantes soit une matière odoriférante isolée. C'est ainsi que le kit peut comprendre plusieurs films comportant chacun une matière odoriférante différente de sorte que plusieurs films mélangés à la composition aqueuse donne un parfum particulier. On peut aussi introduire des parfums ayant des propriétés myorelaxantes, apportant un effet de détente lors de l'application du produit sur la peau.
   Les matières odoriférantes sont des composés usuellement utilisés par les parfumeurs et ils sont notamment décrits dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III. USA.
   Il peut s'agir de produits naturels (huiles essentielles, absolus, résinoïdes, résines, concrètes) et/ou synthétiques (hydrocarbures, alcools, aldéhydes, cétones, éthers, acides, esters, acétals, cétals, nitriles, saturés ou insaturés, aliphatiques ou cycliques).
XIX) Les électrolytes, et notamment les sels des métaux mono-, di- ou tri-valents, et plus particulièrement les sels de métal alcalino-terreux et en particulier les sels de baryum, de calcium et de strontium, les sels de métal alcalin et par exemple les sels de sodium et de potassium, ainsi que les sels de magnésium, de zinc, de manganèse, de fer, de cuivre, d'aluminium, de silicium, de sélénium, et leurs mélanges.
   Les ions constituant ces sels peuvent être choisis par exemple parmi les carbonates, les bicarbonates, les sulfates, les phosphates, les sulfonates, les glycérophosphates, les borates, les bromures, les chlorures, les nitrates, les acétates, les hydroxydes, les persulfates ainsi que les ions d'α-hydroxyacides (citrates, tartrates, lactates, malates) ou d'acides de fruits, les ions d'β-hydroxy-acides (salicylates, hydroxy-2 alcanoates, n-alkyl-salicylates et n-alcanoyl-salicylates), ou encore les ions d'acides aminés (aspartate, arginate, glycocholate, fumarate).
XX) Les ajusteurs de pH et notamment ceux habituellement utilisés dans le domaine cosmétique pour ajuster le pH des compositions à la valeur souhaitée. Il peut s'agir d'acides ou de bases, choisis parmi les bases inorganiques comme l"hydroxyde de sodium, les bases organiques comme les amines (triéthanolamine par exemple), les acides inorganiques comme l'acide chlorhydrique et les acides organiques comme l'acide citrique.

Les composés cités ci-dessus peuvent être présents dans les compositions A et/ou B dans les quantités habituelles dans le domaine considéré, ces quantités dépendant du composé utilisé et du but recherché. Les composés peuvent être présents par exemple en une quantité allant de 0,001 à 30 % en poids, et mieux de 0,01 à 20 % en poids par rapport au poids de la composition A ou de la composition B finale.

Les kit et procédés selon l'invention peuvent être utilisés notamment pour obtenir des produits destinés à être appliqués sur la peau, les muqueuses, les phanères ou les cheveux, notamment comme produits de soin de la peau ou comme produits de maquillage de la peau, de démaquillage ou nettoyage de la peau ou comme produits capillaires ou comme produits solaires.
Selon un mode de réalisation, le kit selon l'invention contient, outre les compositions A et B, au moins une composition additionnelle qui peut comprendre par exemple des actifs cosmétiques tels que cités plus haut.
Dans ce mode de réalisation, le produit final sous forme d'émulsion est de préférence préparé par :
- introduction de la composition additionnelle comprenant les actifs dans la composition B, sous agitation manuelle puis,
- mélange de la composition ci-dessus avec la composition A.

Les exemples ci-après de compositions selon l'invention sont donnés à titre d'illustration et sans caractère limitatif. Les quantités y sont données en % en poids, sauf mention contraire, et les noms sont indiqués en nom chimique ou en nom CTFA selon les cas.

### Exemple 1 : Kit pour produit de soin de la peau

### Composition A

| | |
|---|---|
| Copolymère acrylamide/acrylamido-2-methylpropanesulfonate de sodium en émulsion inverse à 40% dans Polysorbate 80 et isohexadécane (Simulgel 600 de Seppic) | 7,8% |
| Méthylglucose sesquistéarate (Glucate SS de AMERCHOL) | 0,8% |
| Sesquistearate de methylglucoside oxyethyléné (20 OE) (Glucamate SSE-20 de AMERCHOL) | 3,2% |
| Mélange glycérides de palme oxyéthyléné (200OE) et | |
| de coprah oxyéthyléné (7OE) en supension dans eau (55/15/30) (REWODERM LI-S 80 de EVONIK GOLDSCHMIDT) | 5,6% |
| 2-ethylhexyle ether de glycérol (Sensiva SC 50 de Schulke & Mayr) | 0,5% |
| Glycérine | 15,5% |
| Squalane | 18,5% |
| Dicaprylyl carbonate | 15,5% |
| Fraction liquide de beurre de karité | qsp 100% |
| Cire d'abeille | 4% |
| Tocophérols | 1% |

### Mode opératoire

- On chauffe à 60°C sous turbine le dicaprylyle carbonate, la glycerine, le méthylglucose sesquistéarate, le sequistéarate de methylglucoside oxyethyléné (20 OE), le mélange glycérides de palme oxyethyléné (200 OE) et de coprah oxyethyléné (70 OE) et la cire d'abeille jusqu'à solubilisation complète,
- On ajoute à 55 ° C sous turbine, le squalane, la fraction liquide de beurre de karité, le 2-ethylhexyle éther de glycérol et les tocophérols, et on homogénéise l'ensemble.
- On laisse refroidir à 25°C,
- A 25°C, on ajoute sous turbine le copolymère acrylamide/acrylamido-2-methylpropanesulfonate.

### Composition B aqueuse

| | |
|---|---|
| Ethanol | 15 % |
| Eau | qsp 100 % |

Lors de l'utilisation, la consommatrice introduit dans un pot la composition B puis ajoute lentement et sous agitation à l'aide d'une spatule la composition A.

Après quelques secondes d'agitation, une émulsion lisse et régulière se forme.

Le produit ainsi formé peut être conservé au réfrigérateur (pour une durée de 1 à 8 semaines après fabrication).

### Exemple 2 : Kit de soin de la peau

### Composition A

| | |
|---|---|
| Copolymère acrylamide/acrylamido-2-methylpropanesulfonate de sodium en émulsion inverse à 40% dans Polysorbate 80 et isohexadécane (Simulgel 600 de Seppic) | 9% |
| Tri-oléate de sorbitan (NIKKOL SO 30V de NIHON SURFACTANT) | 1,4% |
| Tri-isostéarate de glycéryle polyethoxylé (20 OE) (UNIOX GT 201S L de NOF) | 5,5% |
| Polyethylene sorbitan trioleate (20 OE) (RHEODOL TW - 0320 V de Kao) | 9% |
| 2-ethylhexyle ether de glycérol (Sensiva SC 50 de Schulke & Mayr) | 0,5% |
| Palmitate de dextrine (RHEOPEARL KL-2 OR de Chiba Flour) | 5% |
| Glycérine | 15,5% |
| Squalane | 18,5% |
| Dicaprylyl carbonate | 15,5% |
| Fraction liquide de beurre de karité | qsp 100% |
| Cire d'abeille | 3% |
| Tocophérols | 1% |

### Mode opératoire

- On fait chauffer à 90°C sous turbine le dicaprylyl carbonate, la glycerine, la cire d'abeille, le tri-oléate de sorbitan, le tri-isostéarate de glycéryle polyethoxylé (20 OE) et le polyethylene sorbitan trioleate (20 OE).
- On ajoute le palmitate de dextrine et on homogénéise jusqu'à complète solubilisation
- On refroidit et on maintient quelques minutes la température à 55°C pour homogénéiser,
- On ajoute à 55 ° C sous turbine, le squalane, le beurre de karité, le 2-ethylhexyle éther de glycérol et les tocophérols,
- On refroidit à 25°C

A 25°C, on ajoute sous turbine le copolymère acrylamide/acrylamido-2-ethylpropanesulfonate de sodium.

### Composition B aqueuse

| | |
|---|---|
| 1,3-propanediol | 7 % |
| Ethanol | 15 % |
| Eau | qsp 100 % |

Lors de l'utilisation, la consommatrice introduit dans un pot la composition B puis ajoute lentement et sous agitation à l'aide d'une spatule la composition A.

Après quelques secondes d'agitation, une émulsion lisse et régulière se forme.

Le produit ainsi formé peut être conservé au réfrigérateur (pour une durée de 1 à 8 semaines après fabrication).

### Exemple 3 : Kit

### Composition A

| | |
|---|---|
| Copolymère acrylamide/acrylamido-2-methylpropanesulfonate de sodium en émulsion inverse à 40% dans Polysorbate 80 et isohexadécane (Simulgel 600 de Seppic) | 7,8% |
| Méthylglucose sesquistéarate (Glucamate SS de AMERCHOL) | 0,8% |
| Sesquistearate de methylglucoside oxyethyléné (20 OE) (Glucamate SSE-20 de AMERCHOL) | 3,2% |
| Mélange glycérides de palme oxyéthyléné (200OE) et de coprah oxyéthyléné (7OE) en supension dans eau (55/15/30) (REWODERM LI-S 80 de EVONIK GOLDSCHMIDT) | 5,6% |
| 2-ethylhexyle ether de glycérol (Sensiva SC 50 de Schulke & Mayr) | 0,5% |
| Condensat diacide en C36 hydrogene/ethylene diamine, esterifié par alcool stéarylique (Uniclear 100 VG de Arizona Chemicals) | 10% |
| Glycérine | 15,5% |
| Squalane | 18,5% |
| Dicaprylyl carbonate | 15,5% |
| Fraction liquide de beurre de karité | qsp 100% |
| Tocophérols | 1% |

### Mode opératoire

- On chauffer à 105-110°C sous turbine le dicaprylyl carbonate, la glycerine, le méthylglucose sesquistéarate, le sequistéarate de methylglucoside oxyethyléné (20 OE), le mélange glycérides de palme oxyethyléné (200 OE) et de coprah oxyethyléné (70 OE),
- On ajoute l'Uniclear et on homogénéise jusqu'à complète solubilisation,
- On refroidit et on maintient quelques minutes la température à 55°C pour homogénéiser,,
- On ajoute à 55 ° C sous turbine le squalane, le beurre de karité, le 2-ethylhexyle éther de glycérol et les tocophérols,
- On refroidit à 25°C.
- A 25°C, on ajoute sous turbine le copolymère acrylamide/acrylamido-2-methylpropanesulfonate de sodium.

### Composition B aqueuse

| | |
|---|---|
| 1,3-propanediol | 7,4 % |
| Ethanol | 15 % |
| Eau | qsp 100 % |

Lors de l'utilisation, la consommatrice introduit dans un pot la composition B puis ajoute lentement et sous agitation à l'aide d'une spatule la composition A.

Après quelques secondes d'agitation, une émulsion lisse et régulière se forme.

Le produit ainsi formé peut être conservé au réfrigérateur (pour une durée de 1 à 8 semaines après fabrication).

## Revendications

1. Procédé de traitement cosmétiques des matières kératiniques consistant à :
- disposer d'une composition A comprenant au moins une huile, au moins un agent gélifiant se présentant sous forme dispersée dans un solvant organique ou aqueux, et au moins un tensioactif non ionique choisi parmi les esters de polyols et d'acide gras à chaîne saturée ou insaturée comportant de 8 à 24 atomes de carbone, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre et d'acide gras en C₈-C₂₄, et leurs dérivés oxyalkylénés ; les éthers d'alcools gras ; les éthers de sucre et d'alcools gras en C₈-C₂₄, et leurs mélanges, ladite composition comprenant moins de 10% en poids d'eau
- disposer d'une composition B aqueuse comprenant exclusivement de l'eau et éventuellement au moins un solvant organique hydrosoluble,
- les compositions A et B étant conditionnées séparément,
- mélanger par agitation manuelle de façon extemporanée les compositions A et B de manière à former une émulsion,
puis à appliquer le mélange sur les matières kératiniques.

2. Procédé de traitement cosmétiques des matières kératiniques consistant à :
- disposer d'une composition A comprenant au moins une huile, au moins un agent gélifiant se présentant sous forme dispersée dans un solvant organique ou aqueux, et au moins un tensioactif non ionique choisi parmi les esters de polyols et d'acide gras à chaîne saturée ou insaturée comportant de 8 à 24 atomes de carbone, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés les esters de sucre et d'acide gras en C₈-C₂₄, et leurs dérivés oxyalkylénés ; les éthers d'alcools gras ; les éthers de sucre et d'alcools gras en C₈-C₂₄, et leurs mélanges, ladite composition comprenant moins de 10% en poids d'eau et une teneur en huile allant de 30% à 95% en poids
- disposer d'une composition B aqueuse,
- les compositions A et B étant conditionnées séparément,
- mélanger par agitation manuelle de façon extemporanée les compositions A et B de manière à former une émulsion,
puis à appliquer le mélange sur les matières kératiniques.

3. Procédé selon la revendication 2, dans lequel la composition B comprend exclusivement de l'eau et éventuellement au moins un solvant organique hydrosoluble.

4. Procédé selon l'une quelconque des revendications précédentes, dans lequel la composition B comprend au moins 70%, de préférence au moins 75% et mieux au moins 80% d'eau.

5. Procédé selon la revendication 1, 3 ou 4, dans lequel la composition A présente une teneur en huile allant de 30% à 95% en poids par rapport au poids total de la composition A, de préférence de 40 à 90% en poids, et mieux de 40 à 80% en poids.

6. Procédé selon l'une quelconque des revendications précédentes, dans lequel l'agent gélifiant de la composition A est choisi parmi les polymères se présentant sous forme d'émulsion inverse eau-dans-huile.

7. Procédé selon l'une quelconque des revendications précédentes, dans lequel l'agent gélifiant de la composition A est choisi parmi a) les copolymères d'acide carboxylique à insaturation α,β-monoéthylénique, notamment d'acide acrylique ou méthacrylique, b) les copolymères à base d'acide 2-acrylamido-2-méthylpropane sulfonique et leurs mélanges.

8. Procédé selon l'une quelconque des revendications précédentes, dans lequel la composition A comprend au moins un agent gélifiant choisi parmi les copolymères d'acide 2-acrylamido-2-méthylpropane sulfonique, en particulier les copolymères d'acide 2-acrylamido-2-méthylpropane sulfonique et d'alkyl(méth)acrylamide.

9. Procédé selon l'une quelconque des revendications précédentes, dans lequel l'agent gélifiant est présent en une teneur en matières sèches allant de 0,1% à 25% en poids, de préférence de 0,5% à 20% en poids et encore mieux de 1% à 15% en poids par rapport au poids total de la composition A.

10. Procédé selon l'une quelconque des revendications précédentes dans lequel composition A comprend au moins un tensioactif non ionique choisi parmi les esters de polyols et d'acide gras à chaîne saturée ou insaturée comportant de 12 à 22 atomes de carbone, et leurs dérivés oxyalkylénés tel que les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

11. Procédé selon l'une quelconque des revendications précédentes dans lequel la composition A comprend au moins un agent structurant lipophile choisi parmi : a) les polymères de polyamide, b) les copolymères d'oléfine, c) les polymères semi-cristallins, d) les esters de dextrine et d'acide gras, e) les silices pyrogénées, f) les argiles organophiles et leurs mélanges, les polymères de polyamide sont de formule suivante 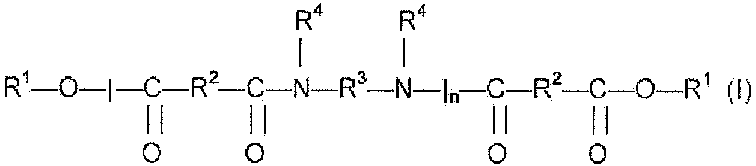 dans laquelle n désigne un nombre entier de motifs amide tel que le nombre de groupes ester représente de 10 % à 50 % du nombre total des groupes ester et amide ; R¹ est indépendamment un groupe alkyle ou alcényle ayant au moins 4 atomes de carbone ; R² représente indépendamment un groupe hydrocarboné en C₄ à C₅₅ à condition que 50 % au moins des groupes R² représentent un groupe hydrocarboné en C₃₀ à C₅₅ ; R³ représente indépendamment un groupe organique pourvu d'au moins 2 atomes de carbone, d'atomes d'hydrogène et optionnellement d'un ou plusieurs atomes d'oxygène ou d'azote ; et R⁴ représente indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

12. Procédé selon l'une quelconque des revendications précédentes dans lequel la composition A comprend au moins un agent structurant lipophile choisi parmi les esters de dextrine et d'acide(s) gras, en particulier le palmitate de dextrine.

13. Procédé selon la revendication 11 ou 12, dans lequel l'agent structurant lipophile représente de 0,5% à 20% en poids, de préférence de 1 % à 15% en poids et encore mieux de 2% à 10% en poids par rapport au poids total de la composition A.

14. Procédé selon l'une quelconque des revendications précédentes dans lequel la composition A comprend moins de 5% en poids d'eau, de préférence moins de 3% en poids d'eau et mieux moins de 1% d'eau.

15. Procédé selon l'une quelconque des revendications précédentes, **caractérisé en ce que** la composition A représente de 2 à 70% en poids, de préférence de 10 à 60% en poids, encore mieux de 20 à 50% en poids par rapport au poids total des compositions A et B.

16. Kit de formulation d'un produit cosmétique sous forme d'émulsion comprenant :
- une composition A comprenant au moins une huile, au moins un agent gélifiant se présentant sous forme dispersée dans un solvant organique ou aqueux et au moins un tensioactif non ionique choisi parmi les esters de polyols et d'acide gras à chaîne saturée ou insaturée comportant de 8 à 24 atomes de carbone, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre et d'acide gras en C₈-C₂₄, et leurs dérivés oxyalkylénés ; les éthers d'alcools gras ; les éthers de sucre et d'alcools gras en C₈-C₂₄, et leurs mélanges, ladite composition comprenant moins de 10% en poids d'eau et une teneur en huile allant de 30% à 95% en poids,
- une composition B aqueuse comprenant exclusivement de l'eau et éventuellement au moins un solvant organique hydrosoluble,
les compositions A et B étant conditionnées séparément.

## Patentansprüche

1. Verfahren zur kosmetischen Behandlung von Keratinsubstanzen, das aus Folgendem besteht:
- man verfügt über eine Zusammensetzung A, umfassend mindestens ein Öl, mindestens ein Geliermittel, das in dispergierter Form in einem organischen oder wässrigen Lösungsmittel vorliegt, und mindestens ein nichtionisches Tensid, ausgewählt aus Estern von Polyolen und Fettsäure mit gesättigter oder ungesättigter Kette mit 8 bis 24 Kohlenstoffatomen und deren oxyalkylenierten Derivaten; Estern von Polyethylenglykol und C₈-C₂₄-Fettsäure und deren oxyalkylenierten Derivaten; Estern von Sorbit und C₈-C₂₄-Fettsäure und deren oxyalkylenierten Derivaten; Estern von Zucker und C₈-C₂₄-Fettsäure und deren oxyalkylenierten Derivaten; Ethern von Fettalkoholen; Ethern von Zucker und C₈-C₂₄-Fettalkoholen und deren Gemischen, wobei die Zusammensetzung weniger als 10 Gew.-% Wasser umfasst
- man verfügt über eine wässrige Zusammensetzung B, die ausschließlich Wasser und gegebenenfalls mindestens ein wasserlösliches organisches Lösungsmittel umfasst,
- wobei die Zusammensetzungen A und B getrennt abgepackt sind,
- man mischt durch manuelles Rühren kurz vor Gebrauch die Zusammensetzungen A und B, um eine Emulsion zu bilden,
dann bringt man das Gemisch auf die Keratinsubstanzen auf.

2. Verfahren zur kosmetischen Behandlung von Keratinsubstanzen, das aus Folgendem besteht:
- man verfügt über eine Zusammensetzung A, umfassend mindestens ein Öl, mindestens ein Geliermittel, das in dispergierter Form in einem organischen oder wässrigen Lösungsmittel vorliegt, und mindestens ein nichtionisches Tensid, ausgewählt aus Estern von Polyolen und Fettsäure mit gesättigter oder ungesättigter Kette mit 8 bis 24 Kohlenstoffatomen und deren oxyalkylenierten Derivaten; Estern von Polyethylenglykol und C₈-C₂₄-Fettsäure und deren oxyalkylenierten Derivaten; Estern von Sorbit und C₈-C₂₄-Fettsäure und deren oxyalkylenierten Derivaten; Estern von Zucker und C₈-C₂₄-Fettsäure und deren oxyalkylenierten Derivaten; Ethern von Fettalkoholen; Ethern von Zucker und C₈-C₂₄-Fettalkoholen und deren Gemischen, wobei die Zusammensetzung weniger als 10 Gew.-% Wasser und einen Ölgehalt von 30 bis 95 Gew.-% umfasst
- man verfügt über eine wässrige Zusammensetzung B,
- wobei die Zusammensetzungen A und B getrennt abgepackt sind,
- man mischt durch manuelles Rühren kurz vor Gebrauch die Zusammensetzungen A und B, um eine Emulsion zu bilden,
dann bringt man das Gemisch auf die Keratinsubstanzen auf.

3. Verfahren nach Anspruch 2, wobei die Zusammensetzung B ausschließlich Wasser und gegebenenfalls mindestens ein wasserlösliches organisches Lösungsmittel umfasst.

4. Verfahren nach einem der vorhergehenden Ansprüche, wobei die Zusammensetzung B mindestens 70%, vorzugsweise mindestens 75% und besser mindestens 80% Wasser umfasst.

5. Verfahren nach Anspruch 1, 3 oder 4, wobei die Zusammensetzung A einen Ölgehalt von 30 bis 95 Gew.-%, bezogen auf das Gesamtgewicht der Zusammensetzung A, vorzugsweise von 40 bis 90 Gew.-% und besser 40 bis 80 Gew.-% aufweist.

6. Verfahren nach einem der vorhergehenden Ansprüche, wobei das Geliermittel der Zusammensetzung A aus Polymeren ausgewählt ist, die in Form einer inversen Wasser-in-Öl-Emulsion vorliegen.

7. Verfahren nach einem der vorhergehenden Ansprüche, wobei das Geliermittel der Zusammensetzung A aus a) Copolymeren von α,β-monoethylenisch ungesättigter Carbonsäure, insbesondere Acryl- oder Methacrylsäure, b) Copolymeren auf der Basis von 2-Acrylamido-2-methylpropansulfonsäure und deren Gemischen ausgewählt ist.

8. Verfahren nach einem der vorhergehenden Ansprüche, wobei die Zusammensetzung A mindestens ein Geliermittel umfasst, ausgewählt aus Copolymeren von 2-Acrylamido-2-methylpropansulfonsäure, insbesondere Copolymeren von 2-Acrylamido-2-methylpropansulfonsäure und Alkyl(meth)acrylamid.

9. Verfahren nach einem der vorhergehenden Ansprüche, wobei das Geliermittel in einem Trockensubstanzgehalt von 0,1 bis 25 Gew.-%, vorzugsweise von 0,5 bis 20 Gew.-% und noch besser von 1 bis 15 Gew.-%, bezogen auf das Gesamtgewicht der Zusammensetzung A, vorliegt.

10. Verfahren nach einem der vorhergehenden Ansprüche, wobei Zusammensetzung A mindestens ein nichtionisches Tensid umfasst, ausgewählt aus Estern von Polyolen und Fettsäure mit gesättigter oder ungesättigter Kette mit 12 bis 22 Kohlenstoffatomen und deren oxyalkylenierten Derivaten, wie Estern von Glyceryl und C₈-C₂₄-Fettsäure und deren oxyalkylenierten Derivaten; Estern von Polyethylenglykol und C₈-C₂₄-Fettsäure und deren oxyalkylenierten Derivaten; Estern von Sorbit und C₈-C₂₄-Fettsäure und deren oxyalkylenierten Derivaten; Estern von Zucker (Saccharose, Glucose, Alkylglucose) und C₈-C₂₄-Fettsäure und deren oxyalkylenierten Derivaten; Ethern von Fettalkoholen; Ethern von Zucker und C₈-C₂₄-Fettalkoholen und deren Gemischen.

11. Verfahren nach einem der vorhergehenden Ansprüche, wobei die Zusammensetzung A mindestens ein lipophiles Strukturierungsmittel umfasst, ausgewählt aus: a) Polyamidpolymeren, b) Olefincopolymeren, c) semikristallinen Polymeren, d) Estern von Dextrin und Fettsäure, e) pyrogenen Kieselsäure, f) organophilen Tonen und deren Gemischen, wobei die Polyamidpolymere die folgende Formel haben: 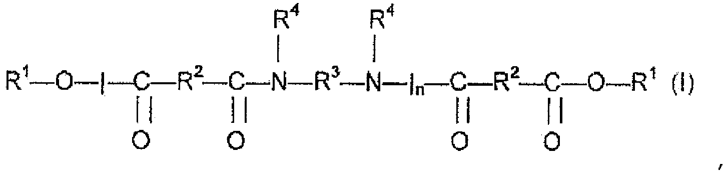 worin n eine ganze Zahl von Amidmotiven bedeutet, so dass die Anzahl der Estergruppen 10% bis 50% der Gesamtanzahl der Ester- und Amidgruppen darstellt; R¹ unabhängig eine Alkyl- oder Alkenylgruppe mit mindestens 4 Kohlenstoffatomen ist; R² unabhängig eine C₄- bis C₅₅-Kohlenwasserstoffgruppe darstellt, mit der Maßgabe, dass mindestens 50% der Gruppen R² eine C₃₀-bis C₅₅-Kohlenwasserstoffgruppe darstellen; R³ unabhängig eine organischen Gruppe mit mindestens 2 Kohlenstoffatomen, Wasserstoffatomen und gegebenenfalls einem oder mehreren Sauerstoff- oder Stickstoffatomen darstellt und R⁴ ein Wasserstoffatom, eine C₁- bis C₁₀-Allylgruppe oder eine direkte Bindung an R³ oder an einen anderen Rest R⁴ darstellt, so dass das Stickstoffatom, an das sowohl R³ als auch R⁴ gebunden sind, Teil einer durch R⁴-N-R³ definierten heterozyklischen Struktur bildet, wobei mindestens 50% der Reste R⁴ ein Wasserstoffatom darstellen.

12. Verfahren nach einem der vorhergehenden Ansprüche, wobei die Zusammensetzung A mindestens ein lipophiles Strukturierungsmittel umfasst, ausgewählt aus Estern von Dextrin und Fettsäure(n), insbesondere Dextrinpalmitat.

13. Verfahren nach Anspruch 11 oder 12, wobei das lipophile Strukturierungsmittel 0,5 bis 20 Gew.-%, vorzugsweise 1 bis 15 Gew.-% und noch besser 2 bis 10 Gew.-%, bezogen auf das Gesamtgewicht der Zusammensetzung A, ausmacht.

14. Verfahren nach einem der vorhergehenden Ansprüche, wobei die Zusammensetzung A weniger als 5 Gew.-% Wasser, vorzugsweise weniger als 3 Gew.-% Wasser und besser weniger als 1% Wasser umfasst.

15. Verfahren nach einem der vorhergehenden Ansprüche, **dadurch gekennzeichnet, dass** die Zusammensetzung A 2 bis 70 Gew.-%, vorzugsweise 10 bis 60 Gew.-%, noch besser 20 bis 50 Gew.-%, bezogen auf das Gesamtgewicht der Zusammensetzungen A und B, ausmacht.

16. Kit zur Formulierung eines kosmetischen Produkts in Form einer Emulsion, umfassend:
- eine Zusammensetzung A, umfassend mindestens ein Öl, mindestens ein Geliermittel, das in dispergierter Form in einem organischen oder wässrigen Lösungsmittel vorliegt, und mindestens ein nichtionisches Tensid, ausgewählt aus Estern von Polyolen und Fettsäure mit gesättigter oder ungesättigter Kette mit 8 bis 24 Kohlenstoffatomen und deren oxyalkylenierten Derivaten; Estern von Polyethylenglykol und C₈-C₂₄-Fettsäure und deren oxyalkylenierten Derivaten; Estern von Sorbit und C₈-C₂₄-Fettsäure und deren oxyalkylenierten Derivaten; Estern von Zucker und C₈-C₂₄-Fettsäure und deren oxyalkylenierten Derivaten; Ethern von Fettalkoholen; Ethern von Zucker und C₈-C₂₄-Fettalkoholen und deren Gemischen, wobei die Zusammensetzung weniger als 10 Gew.-% Wasser und einen Ölgehalt von 30 bis 95 Gew.-% umfasst
- eine wässrige Zusammensetzung B, die ausschließlich Wasser und gegebenenfalls mindestens ein wasserlösliches organisches Lösungsmittel umfasst, wobei die Zusammensetzungen A und B getrennt abgepackt sind.

## Claims

1. Cosmetic process for treating keratin materials, which consists in:
- having a composition A comprising at least one oil, at least one gelling agent in a form dispersed in an organic or aqueous solvent, and at least one nonionic surfactant chosen from esters of polyols and of a fatty acid bearing a saturated or unsaturated chain comprising from 8 to 24 carbon atoms, and oxyalkylenated derivatives thereof; esters of polyethylene glycol and of a C₈-C₂₄ fatty acid, and oxyalkylenated derivatives thereof; esters of sorbitol and of a C₈-C₂₄ fatty acid, and oxyalkylenated derivatives thereof; esters of a sugar and of a C₈-C₂₄ fatty acid, and oxyalkylenated derivatives thereof; fatty alcohol ethers; ethers of a sugar and of C₈-C₂₄ fatty alcohols, and mixtures thereof, said composition comprising less than 10% by weight of water,
- having an aqueous composition B exclusively comprising water and optionally at least one water-soluble organic solvent,
- compositions A and B being packaged separately,
- mixing extemporaneously by manual stirring compositions A and B so as to form an emulsion,
and then applying the mixture to the keratin materials.

2. Cosmetic process for treating keratin materials, which consists in:
- having a composition A comprising at least one oil, at least one gelling agent in a form dispersed in an organic or aqueous solvent, and at least one nonionic surfactant chosen from esters of polyols and of a fatty acid bearing a saturated or unsaturated chain comprising from 8 to 24 carbon atoms, and oxyalkylenated derivatives thereof; esters of polyethylene glycol and of a C₈-C₂₄ fatty acid, and oxyalkylenated derivatives thereof; esters of sorbitol and of a C₈-C₂₄ fatty acid, and oxyalkylenated derivatives thereof; esters of a sugar and of a C₈-C₂₄ fatty acid, and oxyalkylenated derivatives thereof; fatty alcohol ethers; ethers of a sugar and of C₈-C₂₄ fatty alcohols, and mixtures thereof, said composition comprising less than 10% by weight of water and an oil content ranging from 30% to 95% by weight,
- having an aqueous composition B,
- compositions A and B being packaged separately,
- mixing extemporaneously by manual stirring compositions A and B so as to form an emulsion,
and then applying the mixture to the keratin materials.

3. Process according to Claim 2, in which composition B exclusively comprises water and optionally at least one water-soluble organic solvent.

4. Process according to any one of the preceding claims, in which composition B comprises at least 70%, preferably at least 75% and better still at least 80% of water.

5. Process according to Claim 1, 3 or 4, in which composition A has an oil content ranging from 30% to 95% by weight relative to the total weight of composition A, preferably from 40% to 90% by weight and better still from 40% to 80% by weight.

6. Process according to any one of the preceding claims, in which the gelling agent for composition A is chosen from polymers in the form of a water-in-oil reverse emulsion.

7. Process according to any one of the preceding claims, in which the gelling agent for composition A is chosen from a) α,β-monoethylenically unsaturated carboxylic acid copolymers, especially of acrylic or methacrylic acid, b) copolymers based on 2-acrylamido-2-methylpropanesulfonic acid, and mixtures thereof.

8. Process according to any one of the preceding claims, in which composition A comprises at least one gelling agent chosen from 2-acrylamido-2-methylpropanesulfonic acid copolymers, in particular copolymers of 2-acrylamido-2-methylpropanesulfonic acid, and of alkyl(meth)acrylamide.

9. Process according to any one of the preceding claims, in which the gelling agent is in a solids content ranging from 0.1% to 25% by weight, preferably from 0.5% to 20% by weight and better still from 1% to 15% by weight relative to the total weight of composition A.

10. Process according to any one of the preceding claims, in which composition A comprises at least one nonionic surfactant chosen from esters of polyols and of a fatty acid bearing a saturated or unsaturated chain comprising from 12 to 22 carbon atoms, and oxyalkylenated derivatives thereof such as glycerol esters of a C₈-C₂₄ fatty acid, and oxyalkylenated derivatives thereof; esters of polyethylene glycol and of a C₈-C₂₄ fatty acid, and oxyalkylenated derivatives thereof; esters of sorbitol and of a C₈-C₂₄ fatty acid, and oxyalkylenated derivatives thereof; esters of a sugar (sucrose, glucose or an alkylglucose) and of a C₈-C₂₄ fatty acid, and oxyalkylenated derivatives thereof; fatty alcohol ethers; ethers of a sugar and of C₈-C₂₄ fatty alcohols, and mixtures thereof.

11. Process according to any one of the preceding claims, in which composition A comprises at least one lipophilic structuring agent chosen from: a) polyamide polymers, b) olefin copolymers, c) semicrystalline polymers, d) esters of dextrin and of a fatty acid, e) fumed silicas, f) organophilic clays and mixtures thereof, the polyamide polymers having the following formula: 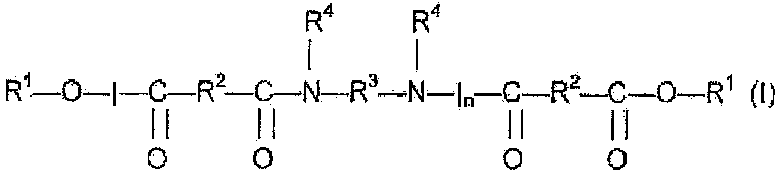 in which n denotes an integer of amide units such that the number of ester groups represents from 10% to 50% of the total number of ester and amide groups; R¹ is, independently, an alkyl or alkenyl group containing at least 4 carbon atoms; R² represents, independently, a C₄ to C₅₅ hydrocarbon-based group on condition that at least 50% of the groups R² represent a C₃₀ to C₅₅ hydrocarbon-based group; R³ represents, independently, an organic group bearing at least two carbon atoms, hydrogen atoms and optionally one or more oxygen or nitrogen atoms; and R⁴ represents, independently, a hydrogen atom, a C₁ to C₁₀ alkyl group or a direct bond to R³ or to another R⁴ such that the nitrogen atom to which both R³ and R⁴ are linked forms part of a heterocyclic structure defined by R⁴-N-R³, with at least 50% of the groups R⁴ representing a hydrogen atom.

12. Process according to any one of the preceding claims, in which composition A comprises at least one lipophilic structuring agent chosen from esters of dextrin and of fatty acid(s), in particular dextrin palmitate.

13. Process according to Claim 11 or 12, in which the lipophilic structuring agent represents from 0.5% to 20% by weight, preferably from 1% to 15% by weight and better still from 2% to 10% by weight relative to the total weight of composition A.

14. Process according to any one of the preceding claims, in which composition A comprises less than 5% by weight of water, preferably less than 3% by weight of water and better still less than 1% of water.

15. Process according to any one of the preceding claims, **characterized in that** composition A represents from 2% to 70% by weight, preferably from 10% to 60% by weight and better still from 20% to 50% by weight relative to the total weight of compositions A and B.

16. Kit for formulation of a cosmetic product in emulsion form, comprising:
- a composition A comprising at least one oil, at least one gelling agent in a form dispersed in an organic or aqueous solvent and at least one nonionic surfactant chosen from esters of polyols and of a fatty acid bearing a saturated or unsaturated chain comprising from 8 to 24 carbon atoms, and oxyalkylenated derivatives thereof; esters of polyethylene glycol and of a C₈-C₂₄ fatty acid, and oxyalkylenated derivatives thereof; esters of sorbitol and of a C₈-C₂₄ fatty acid, and oxyalkylenated derivatives thereof; esters of a sugar and of a C₈-C₂₄ fatty acid, and oxyalkylenated derivatives thereof; fatty alcohol ethers; ethers of a sugar and of C₈-C₂₄ fatty alcohols, and mixtures thereof, said composition comprising less than 10% by weight of water and an oil content ranging from 30% to 95% by weight,
- an aqueous composition B exclusively comprising water and optionally at least one water-soluble organic solvent,
compositions A and B being packaged separately.
